# EUROPEAN PATENT SPECIFICATION

(11) **EP 4 016 534 B1**
(45) Date of publication and mention of the grant of the patent: **08.10.2025**
(21) Application number: 21215684.8
(22) Date of filing: 17.12.2021
(51) Int. Cl.: G16C 60/00, G16C 20/30, G16C 20/70, G06N 3/04

(54) **COMPUTER-IMPLEMENTED METHODS FOR TRAINING A NEURAL NETWORK DEVICE AND CORRESPONDING METHODS FOR GENERATING A FRAGRANCE OR FLAVOR COMPOSITIONS**
COMPUTERIMPLEMENTIERTE VERFAHREN ZUM TRAINIEREN EINER NEURALEN NETZVORRICHTUNG UND ZUGEHÖRIGE VERFAHREN ZUR ERZEUGUNG VON DUFT- ODER AROMAZUSAMMENSETZUNGEN
PROCÉDÉS MIS EN OEUVRE POUR FORMER UN DISPOSITIF DE RÉSEAU NEURONAL ET PROCÉDÉS CORRESPONDANTS DE GÉNÉRATION DE COMPOSITIONS DE PARFUM OU D'ARÔME

(30) Priority: 21.12.2020 EP 20216123; 28.04.2021 EP 21170833
(43) Date of publication of application: 22.06.2022
(73) Proprietor: Firmenich SA, 1242 Satigny (CH)
(72) Inventor: GODIN, Guillaume, 1242 Satigny (CH); VAN DEURSEN, Ruud, 1242 Satigny (CH); NOUCHI, Vincent, 1242 Satigny (CH); CAPELA, Fabio André, 1242 Satigny (CH); RAMET, Gaétan, 1997 Haute-Nendaz (CH); CALVAYRAC, Thibault, 1007 Lausanne (CH); CHICHESTER, Christine, 1242 Satigny (CH)
(74) Representative: dsm-firmenich IP

(56) References cited:
- WO-A1-2020/163860
- XU DAVID: "Machine Learning for Flavor Development", THESIS, 5 April 2019 (2019-04-05), pages 2, 22, 4, 8 - 9, 12, 17,, XP055898228, Retrieved from the Internet <URL:https://dash.harvard.edu/bitstream/handle/1/41940965/ES_100_Final_Report.pdf?sequence=1> [retrieved on 20220307]

## Description

### TECHNICAL FIELD OF THE INVENTION

The present invention relates to a computer-implemented method for training an autoencoder neural network device, a computer-implemented method for training a generative adversarial neural network device and corresponding computer-implemented method for generating a fragrance or flavor composition. The format can be used for predictions and generative design processes in the domain of perfumery and flavor design and manufacturing and more generally to any domain using ingredient compositions, such as pain, gastronomy or medicine.

### BACKGROUND OF THE INVENTION

One way to generate ingredient compositions is to use statistical ingredient usage in an application and apply an optimization technique to determine combinations of ingredient presence and dosage for a given application. This technique is limited to very simple cases like for wine assemblage where the problem dimension is very restricted with fewer than a hundred ingredients maximum.

Current systems, such as the IBM philyra system, works in a similar way by creating a meaningful embedding space that can learn ingredient similarity in a larger ingredient dimension space. However, this system is still close to random, considering that only few percent of generated compositions are deemed as interesting starting points by experts.

In other current systems, random forest neural network devices may be used as a generative tool, in order to obtain pruning or cropping effects on compositions input into the trained random forest neural network device. Such systems are thus unable to generate new compositions in an indeterministic manner.

In other systems, deep belief neural networks may be used as a generative tool. However, such systems require high amounts of postprocessing to filter out viable solutions. Basically, deep belief neural networks explore an entire space to provide solutions that match this space, whether or not those solutions are viable. In turn, this means that deep belief neural networks used in a generative manner are deterministic after training.

In other systems, such as disclosed in WO 2020/163860, olfactive properties of individual molecules are achieved using machine learning technologies. Such systems cannot be used for ingredient composition generation. Furthermore, such systems focus on unitary molecule olfactory property prediction whereas, in reality, the olfactory properties of a composition of ingredients are not linearly linked to the olfactory properties of unitary molecules in said composition. At the very least, a fragrance comprises an ingredient and a solvent, the solvent impacting the olfactory properties of the composition. For instance, the ingredient Indole (registered trademark), more or less diluted, does not present the same smell. As such, there is a composition effect that is not taken into account in such current systems. Xu, David ("Machine Learning for Flavor Development", Bachelor Thesis, Harvard College, 5 April 2019, http:// nrs.harvard.edu/urn-3:HUL.InstRepos:41940965) studied three algorithms (an apriori algorithm, a variational autoencoder and a generative adversarial network) for automating and accelerating flavour development.

The challenge is to be able to scale up the solution to a few thousand possible ingredients, deliver an ingredient composition that is new in terms of composition proximity to existing composition databases and also satisfying multiple criteria optimizations like price, stability, coloration, precipitation, safety, power and performance with reasonable chance to be already good enough in terms of smell and/or taste.

### SUMMARY OF THE INVENTION

The present invention is defined in the appended claims and is intended to remedy all or part of these disadvantages.

To this effect, according to a first aspect, the present invention aims at a computer-implemented method for training an autoencoder neural network or generative adversarial network device to generate indeterministic and realistic digital representations of new fragrance or flavor ingredient compositions to be compounded, characterized in that it comprises the steps of:
- providing an original set of exemplar fragrance or flavor composition digital identifiers, said exemplar fragrance or flavor composition digital identifiers being representative of materialized fragrance or flavor compositions comprising at least two distinct fragrance or flavor compound ingredients, the original set of exemplar fragrance or flavor composition digital identifier further comprising, associated to at least one exemplar fragrance or flavor composition digital identifier, a value representative of at least one hedonic, sensorial and/or physicochemical parameter, and
- training an autoencoder device or generative adversarial network device using the original set of exemplar fragrance or flavor composition digital identifiers to generate a fragrance or flavor composition generative model trained to generate new fragrance or flavor ingredient compositions, comprising at least two distinct fragrance or flavor compound ingredients, to be compounded, as a function of at least one value representative of at least one constraint for generated compositions representative of at least one hedonic, sensorial and/or physicochemical parameter, for the new generated fragrance or flavor composition.

Such provisions allow for the training of generative machine learning devices aimed at the discovery of new compositions or the automatic generation of equivalent compositions that are suited for a different use-case environment than the original composition.

Thanks to these provisions, each time the autoencoder device or generative adversarial network device is used to generate a fragrance or flavor ingredient composition, that fragrance or flavor ingredient composition is new and can reliably be materialized.

In particular embodiments, the value representative of at least one hedonic, sensorial and/or physicochemical parameter, said value being representative of at least one captured hedonic, sensorial and/or physicochemical parameter for the materialized fragrance or flavor composition, is selected from among:
- an olfactive or taste direction associated to the identified fragrance or flavor composition,
- a conditioning medium associated to the identified fragrance or flavor composition,
- a visual and/or olfactive stability or degradation value associated to the identified fragrance or flavor composition,
- a percentage of biodegradability associated to the identified fragrance or flavor composition,
- a percentage of renewable carbon associated to the identified fragrance or flavor composition,
- a perceived psychophysical intensity associated to the identified fragrance or flavor composition,
- a flash point value associated to the identified fragrance or flavor composition,
- a toxicity value associated to the identified fragrance or flavor composition,
- a skin sensitization value associated to the identified fragrance or flavor composition,
- an enhancer compatibility value associated to the identified fragrance or flavor composition,
- a number of ingredients in the composition,
- an environmental accumulation value associated to the identified fragrance or flavor composition and/or
- retention indices for the composition.

The use of such constraints in the step of training allows for the optimization of the capacity of the composition generator to generate compositions within a space that meets certain creation criteria. Such creation criteria are input as constraints both in the training and generating stages.

In particular embodiments, the method object of the present invention comprises a step of capturing a value representative of at least one hedonic, sensorial and/or physicochemical parameter for a least one materialized fragrance or flavor composition, said value being associated to a digital identifier of a fragrance or flavor composition in the exemplar set.

Such embodiments allow for the collection of physical and real-life hedonic, sensorial and/or physicochemical parameters for existing fragrance or flavor compositions. Such collection can then be used in the step of training to ensure that the generated compositions match the generation constraints symbolized by the parameters for which values are captured in the exemplar set.

In particular embodiments:
- the step of providing is configured to provide an original set of exemplar fragrance or flavor composition digital identifiers associated to a primary conditioning medium identifier and at least an additional original set of exemplar fragrance or flavor composition digital identifiers associated to at least one secondary conditioning medium identifier and
- the step of training is configured to train a generative model in which the input is a fragrance or flavor composition digital identifier associated to a primary conditioning medium and the output is a fragrance or flavor composition digital identifier associated to at least one secondary conditioning medium identifier.

Such embodiments allow for the automatic generation of line extensions for fine fragrance, for example. More generally, such embodiments allow for the generation of compositions corresponding to different conditioning applications from a known base application.

In particular embodiments, the step of training is configured to train a variational autoencoder device.

According to a second aspect, the present invention aims at a computer-implemented method for generating a fragrance or flavor composition represented by a digital identifier, comprising a step of generating a fragrance or flavor composition using the trained autoencoder or generative adversarial network device trained according to the training method object of the present invention.

Such provisions allow for the discovery of new compositions or the automatic generation of equivalent compositions that are suited for a different use-case environment than the original composition.

The step of generating is configured to generate a fragrance or flavor composition digital identifier as a function of at least one value representative of at least one input constraint for generated compositions representative of at least one hedonic, sensorial and/or physicochemical parameter, for the generated fragrance or flavor composition digital identifier, among:
- an olfactive or taste direction associated to the identified fragrance or flavor composition,
- a conditioning medium associated to the identified fragrance or flavor composition,
- a visual and/or olfactive stability or degradation value associated to the identified fragrance or flavor composition,
- a percentage of biodegradability associated to the identified fragrance or flavor composition,
- a percentage of renewable carbon associated to the identified fragrance or flavor composition,
- a perceived psychophysical intensity associated to the identified fragrance or flavor composition,
- a flash point value associated to the identified fragrance or flavor composition,
- a toxicity value associated to the identified fragrance or flavor composition,
- a skin sensitization value associated to the identified fragrance or flavor composition,
- an enhancer compatibility value associated to the identified fragrance or flavor composition,
- a number of ingredients in the composition,
- an environmental accumulation value associated to the identified fragrance or flavor composition and/or
- retention indices for the composition.

Such embodiments allow the specification of generation targets to be reached by the use of the machine learning devices.

**In** particular embodiments, the step of generating is configured to generate a fragrance or flavor composition digital identifier associated to at least one secondary conditioning medium identifier as a function of an input of at least one fragrance or flavor composition digital identifier associated to a primary conditioning medium identifier.

Such embodiments allow for the automatic generation of line extensions for fine fragrance, for example. More generally, such embodiments allow for the generation of compositions corresponding to different conditioning applications from a known base application.

**In** particular embodiments, the method object of the present invention comprises:
- a step of generating a trained auxiliary machine learning device, comprising:
   - providing an original set of exemplar fragrance or flavor composition digital identifiers and labels representative of a chemical feature, such as an olfaction feature value or a value representative of chemical compound quantity associated to said structure,
   - training the auxiliary machine learning device using the original set of exemplar fragrance or flavor composition digital identifiers to provide a composition classifier and
- a step of constraining the step of training a generative adversarial network device or an autoencoder device with the machine learning device, said step being executed during said step of training, as a reinforcement rule, or via postprocessing of the fragrance or flavor composition identifier generated.

Such embodiments allow for the generation of compositions under constraints resulting from the training of other, specialized, machine learning devices.

**In** particular embodiments, any method object of the present invention further comprises a step of compounding a generated fragrance or flavor composition comprising at least two distinct ingredients.

This step of compounding allows for the generated fragrance or flavor composition to be obtained in a physical manner, similarly to the way a perfumer would generate a fragrance or flavor which would then be obtained according to any relevant chemical process.

In particular embodiments, the method object of the present invention further comprises a step of selecting a generated fragrance or flavor composition, comprising at least two distinct ingredients, to be compounded.

Such embodiments allow for the manual or automatic selection of generated fragrance or flavor composition to be compounded, or produced, and be distributed.

According to a third aspect, the present invention aims at a computer-implemented autoencoder device trained according to the method object of the present invention.

According to a fourth aspect, the present invention aims at a computer-implemented generative adversarial network device trained according to the method object of the present invention.

According to a fifth aspect, the present invention aims at a computer program product comprising instructions which, when the program is executed by a computer, cause the computer to carry out any method object of the present invention.

According to a sixth aspect, the present invention aims at a computer-readable storage medium storing instructions which, when executed by a computer, cause the computer to carry out the steps of any method object of the present invention.

According to a seventh aspect, the present invention aims at a device for training an autoencoder neural network or generative adversarial network device to generate indeterministic and realistic digital representations of new fragrance or flavor ingredient compositions to be compounded, comprising the steps of:
- a means of providing an original set of exemplar fragrance or flavor composition digital identifiers, said exemplar fragrance or flavor composition digital identifiers being representative of materialized fragrance or flavor compositions comprising at least two distinct fragrance or flavor compound ingredients, the original set of exemplar fragrance or flavor composition digital identifier further comprising, associated to at least one exemplar fragrance or flavor composition digital identifier, a value representative of at least one hedonic, sensorial and/or physicochemical parameter, and
- a means of training an autoencoder device or generative adversarial network device using the original set of exemplar fragrance or flavor composition digital identifiers to generate a fragrance or flavor composition generative model trained to generate new fragrance or flavor ingredient compositions, comprising at least two distinct fragrance or flavor compound ingredients, to be compounded, as a function of at least one value representative of at least one constraint for generated compositions representative of at least one hedonic, sensorial and/or physicochemical parameter, for the new generated fragrance or flavor composition.

According to an eighth aspect, the present invention aims at a device for generating a fragrance or flavor composition digital identifier, comprising a means of generating a fragrance or flavor composition using the trained autoencoder or generative adversarial network device trained according to the method object of the present invention.

The third to eighth aspects of the present invention exhibit the same advantages as the related first and second aspects.

### BRIEF DESCRIPTION OF THE DRAWINGS

Other advantages, purposes and particular characteristics of the invention shall be apparent from the following non-exhaustive description of at least one particular embodiment of the present invention, in relation to the drawings annexed hereto, in which:
[Figure 1] represents, schematically, a first succession of steps of a particular embodiment of the method object of the present invention,
[Figure 2] represents, schematically, a second succession of steps of a particular embodiment of the method object of the present invention,
[Figure 3] represents, schematically, a third succession of steps of a particular embodiment of the method object of the present invention,
[Figure 4] represents, schematically, a first particular embodiment of the device object of the present invention,
[Figure 5] represents, schematically, a second particular embodiment of the device object of the present invention,
[Figure 6] represents, schematically, a third particular embodiment of the device object of the present invention,
[Figure 7] represents, schematically, a fourth particular embodiment of the device object of the present invention,
[Figure 8] represents, schematically, a fifth particular embodiment of the device object of the present invention,
[Figure 9] represents, schematically, a sixth particular embodiment of the device object of the present invention,
[Figure 10] represents, schematically, a seventh particular embodiment of the device object of the present invention,
[Figure 11] represents, schematically, an eighth particular embodiment of the device object of the present invention and
[Figure 12] represents, schematically, a fourth succession of steps of a particular embodiment of the method object of the present invention.

### DETAILED DESCRIPTION OF THE INVENTION

This description is not exhaustive, as each feature of one embodiment may be combined with any other feature of any other embodiment in an advantageous manner. Also, various inventive concepts may be embodied as one or more methods, of which an example has been provided. The acts performed as part of the method may be ordered in any suitable way. Accordingly, embodiments may be constructed in which acts are performed in an order different than illustrated, which may include performing some acts simultaneously, even though shown as sequential acts in illustrative embodiments.

The indefinite articles 'a' and 'an', as used herein in the specification and in the claims, unless clearly indicated to the contrary, should be understood to mean 'at least one'.

The phrase 'and/or', as used herein in the specification and in the claims, should be understood to mean 'either or both' of the elements so conjoined, i.e. elements that are conjunctively present in some cases and disjunctively present in other cases. Multiple elements listed with 'and/or' should be construed in the same fashion, i.e. 'one or more' of the elements so conjoined. Other elements may optionally be present other than the elements specifically identified by the 'and/or' clause whether related or unrelated to those elements specifically identified. Thus, as a non-limiting example, a reference to 'A and/or B', when used in conjunction with open-ended language such as 'comprising' can refer, in one embodiment, to A only (optionally including elements other than B); in another embodiment, to B only (optionally including elements other than A); in yet another embodiment, to both A and B (optionally including other elements); etc.

As used herein in the specification and in the claims, 'or' should be understood to have the same meaning as 'and/or' as defined above. For example, when separating items in a list, 'or' or 'and/or' shall be interpreted as being inclusive, i.e. the inclusion of at least one, but also including more than one, of a number or list of elements, and, optionally, additional unlisted items. Only terms clearly indicated to the contrary, such as 'only one of' or 'exactly one of', or, when used in the claims, 'consisting of', will refer to the inclusion of exactly one element of a number or list of elements. In general, the term 'or' as used herein shall only be interpreted as indicating exclusive alternatives (i.e. 'one or the other but not both') when preceded by terms of exclusivity, such as 'either,' 'one of,' 'only one of', or 'exactly one of'. 'Consisting essentially of,' when used in the claims, shall have its ordinary meaning as used in the field of patent law.

As used herein in the specification and in the claims, the phrase 'at least one', in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase 'at least one' refers, whether related or unrelated to those elements specifically identified. Thus, as a non-limiting example, 'at least one of A and B' (or, equivalently, 'at least one of A or B', or, equivalently 'at least one of A and/or B') can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

In the claims, as well as in the specification above, all transitional phrases such as 'comprising,' 'including,' 'carrying,' 'having,' 'containing,' 'involving,' 'holding,' 'composed of', and the like are to be understood to be open-ended, i.e. to mean including but not limited to. Only the transitional phrases 'consisting of' and 'consisting essentially of' shall be closed or semi-closed transitional phrases, respectively.

It should be noted at this point that the figures are not to scale.

Perception of flavors and fragrances is driven by the interactions between the chemical mixture of a product and the biological receptors of an individual. From the chemical product to the perception, a highly non-linear chain of processes is taking place to create sensations and emotions that determine the positive/negative feedback of the final product.

Deep neural networks have already allowed to model several aspects of the human cognition such as vision, hearing and language understanding. Such models have also been used on the generation of very realistic images and sounds/songs. However, models on the olfaction and taste have received very little attention, mainly because of the limited amount of data available.

The aim of the models presented herein is to generate new alternative fragrance or flavor compositions by considering certain targets, such as:
- an olfactive or taste direction associated to the identified fragrance or flavor composition such as 'Green, Woody, Vanilla' or 'Fruity, Citrus, Aromatic', 'Roasted Chicken', 'Strawberry, Citrus' olfactive directions, a taste direction corresponding in this case, to a descriptor of the taste or smell of a fragrance or flavor, such a descriptor is representative of the taste or smell of another item that is similar or close in terms of psychochemical perception, such a descriptor is sometimes known as the character of an ingredient,
- a conditioning medium associated to the identified fragrance or flavor composition, such a conditioning medium being associated to a fragrance or flavor application of a consumer product where the identified fragrance or flavor composition is put in; such a conditioning medium designates a chemical base allowing for the transportation of a fragrant molecule. Examples of applications are: 'Soap Bar', 'Detergent Liquid', 'Detergent Powder', 'Shampoo', 'Skin Care/Face', 'Beverages',' Soups & Stocks', 'Sweets', 'Desserts', 'Diary', etc.,
- a visual and/or olfactive/taste stability assessment control associated to the identified fragrance or flavor composition in a consumer product: no precipitate very slightly yellow coloration, very slightly odor variation, such a value corresponding to a score or percentage representative of the stability of the fragrance or flavor,
- a percentage of biodegradability associated to the identified fragrance or flavor composition; such a unit can be measured as a percentage, such as for example 95% of the chemicals proportion in the composition is biodegradable in tests after 28 days,
- a percentage of renewable carbon associated to the identified fragrance or flavor composition; such a unit can be measured as a percentage, such as, for example 65% of the chemicals proportion in the composition are renewable carbon sourced and not originating from petrochemical sourcing but rather from natural sourcing or from a chemical or biological molecule transformed while maintaining a large part of renewable carbon criteria (more than 50% of carbons do not originate from petrochemical sourcing),
- a perceived psychophysical intensity associated to the identified fragrance or flavor composition; such a unit can be measured as a percentage such as, for example, 64% in a reference scale of odor intensity,
- a flash point value associated to the identified fragrance or flavor composition; such a unit can be measured as a percentage such as, for example, 67 °C allowing to transport the fragrance by plane or ship without risk of explosion,
- a toxicity value associated to the identified fragrance or flavor composition; such a unit can be measured as a percentage such as, for example, 100% of chemicals in the composition are nontoxic,
- a skin sensitization value associated to the identified fragrance/flavor composition,
- a value representative of a diet type, such as gluten-free or vegetarian for example,
- a number of ingredients in the composition,
- an enhancer compatibility value associated to the identified fragrance or flavor composition; such a unit can be measured as a percentage such as, for example, like sucralose percentage, fructose percentage, umami percentage, enhancer percentage,
- an environmental accumulation value associated to the identified fragrance or flavor composition and/or
- retention indices for the composition.

The terms 'fragrance or flavor composition digital identifier' refer to any digital representation of a fragrance or flavor composition. Such a composition corresponds to an association of at least two ingredients. The corresponding digital representation may correspond to an entry identifier in a database of a graph or linear notation of the composition, for example. Such a linear notation may correspond to a SMILES (for 'Simplified Molecular Input Line Entry Specification') string, for example. Alternatively, such a composition may be referenced to by an alphanumeric name, corresponding to an ingredient name.

As used herein, the terms 'computing system' designate any electronic calculation device, whether unitary or distributed, capable of receiving numerical inputs and providing numerical outputs by and to any sort of interface, such as a digital interface. Typically, a computing system designates either a computer executing a software having access to data storage or a client-server architecture wherein the data and/or calculation is performed at the server side while the client side acts as an interface.

It should be understood that one of the key advantages of the present invention is the capacity of the trained autoencoder device or generative adversarial network device to generate indeterministic composition identifiers. The term 'indeterministic' refers to the capacity of the autoencoder device or generative adversarial network device to generate truly random composition identifiers that are realistically representative of new unmaterialized composition identifiers to be materialized through compounding. In other words, indeterministic systems do not replicate or derive values associated to the original, materialized, composition identifiers but rather create entirely new composition identifiers. Such an indeterministic capacity is also represented by the fact that the autoencoder device or generative adversarial network device can be trained to generate a multitude of composition identifiers based upon a single input. In such systems, for example, the latent space defines an input applicability domain known as a convex hull. An input vector is selected from within this domain. The use of generation constraints in the input modifies the applicability domain, such that the input, extracted from said applicability domain, differs from one set of constraints to another.

By 'realistic' representations, it is intended that the generated composition identifiers are representative of material world behavior for corresponding materialized compositions. This means, for example, that if a generative adversarial network has been trained using an original set of composition identifiers associated with particular sensorial olfactive or taste directions, then the generative adversarial network will generate composition identifiers that match the initial sensorial olfactive or taste directions of the original set, thus being representative of the material world.

In the present description, the term 'materialized' is intended as existing outside of the digital environment of the present invention. 'Materialized' may mean, for example, readily found in nature or synthesized in a laboratory or chemical plant. In any event, a materialized composition presents a tangible reality. The terms 'to be compounded' or 'compounding' refer to the act of materialization of a composition, whether via extraction and assembly of ingredients or via synthetization and assembly of ingredients.

As used herein, the terms 'ingredient' designates a perfuming ingredient, a flavor ingredient, a perfumery carrier, a flavor carrier, a perfumery adjuvant, a flavor adjuvant, a perfumery modulator, flavor modulator. Preferably, such a fragrance or fragrant chemical compound is volatile. Such an ingredient may be a natural ingredient.

By 'perfuming ingredient' it is meant here a compound, which is used in a perfuming preparation or a composition to impart a hedonic effect. In other words, such an ingredient, to be considered as being a perfuming one, must be recognized by a person skilled in the art as being able to impart or modify in a positive or pleasant way the odor of a composition, and not just as having an odor.

The nature and type of the perfuming ingredients do not warrant a more detailed description here, which in any case would not be exhaustive, the skilled person being able to select them on the basis of his general knowledge and according to the intended use or application and the desired organoleptic effect. In general terms, these perfuming co-ingredients belong to chemical classes as varied as alcohols, lactones, aldehydes, ketones, esters, ethers, acetates, nitriles, terpenoids, nitrogenous or sulphurous heterocyclic compounds and essential oils, and said perfuming co-ingredients can be of natural or synthetic origin. Said perfuming ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of perfumery. It is also understood that said perfuming ingredients may also be compounds known to release in a controlled manner various types of perfuming ingredients also known as properfume or profragrance.

By 'perfumery carrier' it is meant here a material which is practically neutral from a perfumery point of view, i.e. that does not significantly alter the organoleptic properties of perfuming ingredients. Said carrier may be a liquid or a solid.

As liquid carrier one may cite, as non-limiting examples, an emulsifying system, i.e. a solvent and a surfactant system, or a solvent commonly used in perfumery. A detailed description of the nature and type of solvents commonly used in perfumery cannot be exhaustive. However, one can cite as non-limiting examples, solvents such as butylene or propylene glycol, glycerol, dipropyleneglycol and its monoether, 1,2,3-propanetriyl triacetate, dimethyl glutarate, dimethyl adipate 1,3-diacetyloxypropan-2-yl acetate, diethyl phthalate, isopropyl myristate, benzyl benzoate, benzyl alcohol, 2-(2-ethoxyethoxy)-1-ethano, tri-ethyl citrate or mixtures thereof, which are the most commonly used. For the compositions which comprise both a perfumery carrier and a perfumery base, other suitable perfumery carriers than those previously specified, can be also ethanol, water/ethanol mixtures, limonene or other terpenes, isoparaffins such as those known under the trademark Isopar^{®} (origin: Exxon Chemical) or glycol ethers and glycol ether esters such as those known under the trademark Dowanol^{®} (origin: Dow Chemical Company), or hydrogenated castors oils such as those known under the trademark Cremophor^{®} RH 40 (origin: BASF).

Solid carrier is meant to designate a material to which the perfuming composition or some element of the perfuming composition can be chemically or physically bound. In general such solid carriers are employed either to stabilize the composition, or to control the rate of evaporation of the compositions or of some ingredients. The use of solid carriers is of current use in the art and a person skilled in the art knows how to reach the desired effect. However, by way of non-limiting examples of solid carriers, one may cite absorbing gums or polymers or inorganic materials, such as porous polymers, cyclodextrins, wood based materials, organic or inorganic gels, clays, gypsum talc or zeolites.

As other non-limiting examples of solid carriers, one may cite encapsulating materials. Examples of such materials may comprise wall-forming and plasticizing materials, such as mono, di- or trisaccharides, natural or modified starches, hydrocolloids, cellulose derivatives, polyvinyl acetates, polyvinylalcohols, proteins or pectins, or yet the materials cited in reference texts such as H. Scherz, Hydrokolloide: Stabilisatoren, Dickungs - und Geliermittel in Lebensmitteln, Band 2 der Schriftenreihe Lebensmittelchemie, Lebensmittelqualität, Behr's Verlag GmbH & Co., Hamburg, 1996. The encapsulation is a well-known process to a person skilled in the art, and may be performed, for instance, by using techniques such as spray-drying, agglomeration or yet extrusion; or consists of a coating encapsulation, including coacervation and complex coacervation techniques.

As non-limiting examples of solid carriers, one may cite in particular the core-shell capsules with resins of aminoplast, polyamide, polyester, polyurea or polyurethane type or a mixture threof (all of said resins are well known to a person skilled in the art) using techniques like phase separation process induced by polymerization, interfacial polymerization, coacervation or altogether (all of said techniques have been described in the prior art), optionally in the presence of a polymeric stabilizer or of a cationic copolymer.

Resins may be produced by the polycondensation of an aldehyde (e.g. formaldehyde, 2,2-dimethoxyethanal, glyoxal, glyoxylic acid or glycolaldehyde and mixtures thereof) with an amine such as urea, benzoguanamine, glycoluryl, melamine, methylol melamine, methylated methylol melamine, guanazole and the like, as well as mixtures thereof. Alternatively, one may use preformed resins alkylolated polyamines such as those commercially available under the trademark Urac^{®} (origin: Cytec Technology Corp.), Cymel^{®} (origin: Cytec Technology Corp.), Urecoll^{®} or Luracoll^{®} (origin: BASF).

Others resins one are the ones produced by the polycondensation of an a polyol, like glycerol, and a polyisocyanate, like a trimer of hexamethylene diisocyanate, a trimer of isophorone diisocyanate or xylylene diisocyanate or a Biuret of hexamethylene diisocyanate or a trimer of xylylene diisocyanate with trimethylolpropane (known with the tradename of Takenate^{®}, origin: Mitsui Chemicals), among which a trimer of xylylene diisocyanate with trimethylolpropane and a Biuret of hexamethylene diisocyanate are preferred.

Some of the seminal literature related to the encapsulation of perfumes by polycondensation of amino resins, namely melamine-based resins with aldehydes includes represented by articles such as those published by K. Dietrich et al. Acta Polymerica, 1989, vol. 40, pages 243, 325 and 683, as well as 1990, vol. 41, page 91. Such articles already describe the various parameters affecting the preparation of such core-shell microcapsules following prior art methods that are also further detailed and exemplified in the patent literature. US 4'396'670, to the Wiggins Teape Group Limited is a pertinent early example of the latter. Since then, many other authors have enriched the literature in this field and it would be impossible to cover all published developments here, but the general knowledge in encapsulation technology is very significant. More recent publications of pertinencypertinence, which disclose suitable uses of such microcapsules, are represented for example by the article of K. Bruyninckx and M. Dusselier, ACS Sustainable Chemistry & Engineering, 2019, vol. 7, pages 8041-8054H.Y.Lee et al. Journal of Microencapsulation, 2002, vol. 19, pages 559-569, international patent publication WO 01/41915 or yet the article of S. Bône et al. Chimia, 2011, vol. 65, pages 177-181.

By 'perfumery adjuvant', it is meant here an ingredient capable of imparting additional added benefit such as a color, a particular light resistance, chemical stability, etc. A detailed description of the nature and type of adjuvant commonly used in perfuming composition cannot be exhaustive, but it has to be mentioned that said ingredients are well known to a person skilled in the art. One may cite as specific non-limiting examples the following: viscosity agents (e.g. surfactants, thickeners, gelling and/or rheology modifiers), stabilizing agents (e.g. preservatives, antioxidant, heat/light and or buffers or chelating agents, such as BHT), coloring agents (e.g. dyes and/or pigments), preservatives (e.g. antibacterial or antimicrobial or antifungal or anti irritant agents), abrasives, skin cooling agents, fixatives, insect repellants, ointments, vitamins and mixtures thereof.

By 'perfumery modulator', it is understood here an agent having the capacity to affect the manner in which the odour, and in particular the evaporation rate and intensity, of the compositions incorporating said modulator can be perceived by an observer or user thereof, over time, as compared to the same perception in the absence of the modulator. Perfumery modulators are also known as fixative. In particular, the modulator allows prolonging the time during which their fragrance is perceived. Non-limiting examples of suitable modulators may include methyl glucoside polyol; ethyl glucoside polyol; propyl glucoside polyol; isocetyl alcohol; PPG-3 myristyl ether; neopentyl glycol diethylhexanoate; sucrose laurate; sucrose dilaurate, sucrose myristate, sucrose palmitate, sucrose stearate, sucrose distearate, sucrose tristearate, hyaluronic acid disaccharide sodium salt, sodium hyaluronate, propylene glycol propyl ether; dicetyl ether; polyglycerin-4 ethers; isoceteth-5; isoceteth-7, isoceteth-10; isoceteth-12; isoceteth-15; isoceteth-20; isoceteth-25; isoceteth-30; disodium lauroamphodipropionate; hexaethylene glycol monododecyl ether; and their mixtures; neopentyl glycol diisononanoate; cetearyl ethylhexanoate ; panthenol ethyl ether, DL-panthenol, N-hexadecyl n-nonanoate, noctadecyl n-nonanoate, a profragrance, cyclodextrin, an encapsulation, and a combination thereof.

By 'flavoring ingredient' it is meant here a compound, which is used in flavoring preparations or compositions to impart a hedonic effect. In other words, such an ingredient, to be considered as being a flavoring one, must be recognized by a person skilled in the art as being able to impart or modify in a positive or pleasant way the taste of a composition, and not just as having a taste. The nature and type of the flavoring ingredients present in the composition do not warrant a more detailed description here, the skilled person being able to select them on the basis of its general knowledge and according to intended use or application and the desired organoleptic effect. In general terms, these flavoring ingredients belong to chemical classes as varied as alcohols, aldehydes, ketones, esters, ethers, acetates, nitriles, terpenoids, nitrogenous or sulphurous heterocyclic compounds and essential oils, and said flavoring ingredients can be of natural or synthetic origin. Many of these ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of flavor. It is also understood that said co-ingredients may also be compounds known to release in a controlled manner various types of flavoring compounds, also called pro-flavor.

The term 'flavor carrier' designates a material which is substantially neutral from a flavor point of view, insofar as it does not significantly alter the organoleptic properties of flavoring ingredients. The carrier may be a liquid or a solid.

Suitable liquid carriers include, for instance, an emulsifying system, i.e. a solvent and a surfactant system, or a solvent commonly used in flavors. A detailed description of the nature and type of solvents commonly used in flavor cannot be exhaustive. Suitable solvents used in flavor include, for instance, propylene glycol, triacetine, caprylic/capric triglyceride (neobee^{®}), triethyl citrate, benzylic alcohol, ethanol, isopropanol, citrus terpenes, vegetable oils such as Linseed oil, sunflower oil or coconut oil, glycerol.

Suitable solid carriers include, for instance, absorbing gums or polymers, or even encapsulating materials. Examples of such materials may comprise wall-forming and plasticizing materials, such as mono, di- or polysaccharides, natural or modified starches, hydrocolloids, cellulose derivatives, polyvinyl acetates, polyvinylalcohols, xanthan gum, arabic gum, acacia gum or yet the materials cited in reference texts such as H. Scherz, Hydrokolloid: Stabilisatoren, Dickungs - und Geliermittel in Lebensmitteln, Band 2 der Schriftenreihe Lebensmittelchemie, Lebensmittelqualität, Behr's VerlagGmbH & Co., Hamburg, 1996. Encapsulation is a well-known process to a person skilled in the art, and may be performed, for instance, using techniques such as spray-drying, agglomeration, extrusion, coating, plating, coacervation and the like.

By 'flavor adjuvant', it is meant here an ingredient capable of imparting additional added benefit such as a color (e.g. caramel), chemical stability, and so on. A detailed description of the nature and type of adjuvant commonly used in flavoring compositions cannot be exhaustive. Nevertheless, such adjuvants are well known to a person skilled in the art who will be able to select them on the basis of its general knowledge and according to intended use or application. One may cite as specific non-limiting examples the following: viscosity agents (e.g. emulsifier, thickeners, gelling and/or rheology modifiers, e.g. pectin or agar gum), stabilizing agents (e.g. antioxidant, heat/light and or buffers agents e.g. citric acid), coloring agents (e.g. natural or synthetic or natural extract imparting color), preservatives (e.g. antibacterial or antimicrobial or antifungal agents, e.g. benzoic acid), vitamins and mixtures thereof.

By 'flavor modulator', it is meant here an ingredient capable to enhance sweetness, to block bitterness, to enhance umami, to reduce sourness or licorice taste, to enhance saltiness, to enhance a cooling effect, or any combinations of the foregoing. Flavor modulators are also called trigeminal sensates.

As used herein, the term 'composition' designates a liquid, solid or gaseous assembly of at least one volatile molecule.

As used herein, the term 'fragrance' refers to the olfactory perception resulting from the sum of odorant receptor activation, enhancement, and inhibition by at least one fragrant chemical compound.

As used herein, the term 'flavor' refers to the olfactory and/or taste perception resulting from the sum of olfative and/or taste receptor activation, enhancement, and inhibition by at least ingredient in the composition..

As used herein, the terms 'exemplar fragrance or flavor composition digital identifier' refers to the digitized representation of a materialized fragrance or flavor composition.

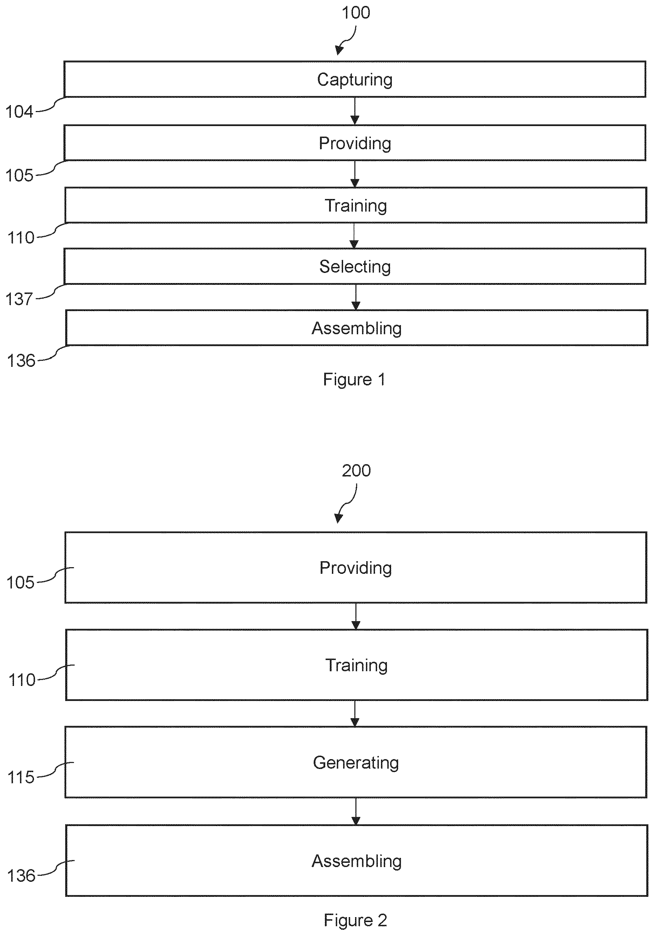

Figure 1 shows a succession of steps of a particular embodiment of the method 100 object of the present invention. This computer-implemented method 100 for training an autoencoder neural network or generative adversarial network device to generate indeterministic and realistic digital representations of new fragrance or flavor ingredient compositions to be compounded, comprises the steps of:
- providing 105 an original set of exemplar fragrance or flavor composition digital identifiers, said exemplar fragrance or flavor composition digital identifiers being representative of materialized fragrance or flavor compositions comprising at least two distinct ingredients and
- training 110 an autoencoder device or generative adversarial network device using the original set of exemplar fragrance or flavor composition digital identifiers to generate a fragrance or flavor composition generative model trained to generate new fragrance or flavor ingredient compositions, comprising at least two distinct ingredients, to be compounded.

The step of providing 105 is performed, for example, by a computer program executed by an electronic computation device, such as a microprocessor. During this step of providing 105, at least one exemplar fragrance or flavor composition digital identifier is provided to the machine learning device to be trained. Such a step of providing 105 may correspond to the transfer in memory of the exemplar fragrance or flavor composition digital identifier from a digital storage location to another, the latter being dedicated to the training set of the machine learning device. During such a step of providing 105, a larger sample of fragrance or flavor composition digital identifiers representative of distinct fragrance or flavor compositions may be divided into a training set and into a validation set.

The step of training 110 may use any training method appropriated for training an autoencoder device or generative adversarial network device.

In particular embodiments, the original set of exemplar fragrance further comprises, associated to at least one exemplar fragrance or flavor composition digital identifier, a value representative of at least one hedonic, sensorial and/or physicochemical parameter, said value being representative of at least one captured hedonic, sensorial and/or physicochemical parameter for the materialized fragrance or flavor composition, among:
- an olfactive or taste direction associated to the identified fragrance or flavor composition,
- a conditioning medium associated to the identified fragrance or flavor composition,
- a visual and/or olfactive stability or degradation value associated to the identified fragrance or flavor composition,
- a percentage of biodegradability associated to the identified fragrance or flavor composition,
- a percentage of renewable carbon associated to the identified fragrance or flavor composition,
- a perceived psychophysical intensity associated to the identified fragrance or flavor composition,
- a flash point value associated to the identified fragrance or flavor composition,
- a toxicity value associated to the identified fragrance or flavor composition,
- a skin sensitization value associated to the identified fragrance or flavor composition,
- an enhancer compatibility value associated to the identified fragrance or flavor composition,
- a number of ingredients in the composition,
- an environmental accumulation value associated to the identified fragrance or flavor composition and/or
- retention indices for the composition, such as the Kovats retention index..

**In** particular embodiments, such as shown in figure 1, the present invention comprises a step 104 of capturing a value representative of at least one hedonic, sensorial and/or physicochemical parameter for a least one materialized fragrance or flavor composition, said value being associated to a digital identifier of a fragrance or flavor composition in the exemplar set.

Such a step 104 of capturing may be performed, for example, by using any type of sensor fitting the hedonic, sensorial and/or physicochemical parameter to be captured. For example, skin sensitization can be measured with anti-bac skin assays or LLNA method.

**In** particular embodiments:
- the step 105 of providing is configured to provide an original set of exemplar fragrance or flavor composition digital identifiers associated to a primary conditioning medium identifier and at least an additional original set of exemplar fragrance or flavor composition digital identifiers associated to at least one secondary conditioning medium identifier,
- the step 110 of training is configured to train a generative model in which the input is a fragrance or flavor composition digital identifier associated to a primary conditioning medium and the output is a fragrance or flavor composition digital identifier associated to at least one secondary conditioning medium identifier.

An example of primary conditioning medium is 'Soap Bar' with an olfactive direction being 'Lily of the valley, Gourmand, Floral', a carbon renewability percentage of 70% and biodegradability of 100%.

An example of the step 110 of training can have a fragrance or flavor composition digital identifier associated with a conditioning medium of 'Soups & Stocks' and an olfactive profile of 'Roasted Chicken' and the output could be a conditioning medium of 'Beverages' with an olfactive profile of 'Citrus' end product is a lemonade soda.

A conditioning medium, or transport medium, may correspond to a chemical base that holds the fragrant molecules until release, such as a solvent for perfumes or a surfactant base for liquid soap or shampoo applications. Such a transport medium may also correspond to an intended application of the fragrance or flavor composition, such as a fine fragrance, body care, home care, food product or aroma additive application.

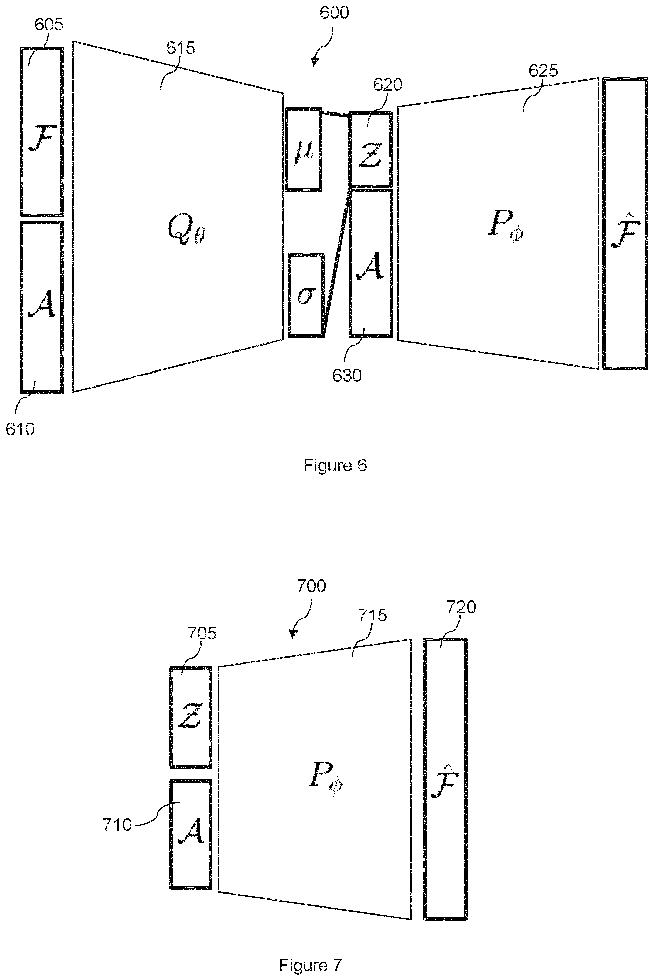

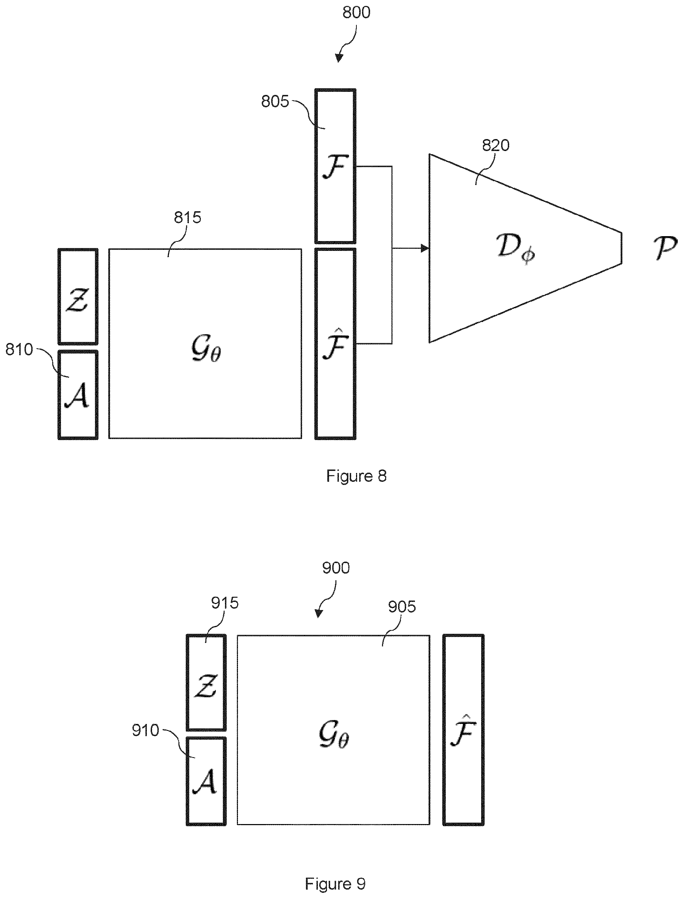

Below, two examples are presented:
- a first example related to the training of an autoencoder device 600 illustrated by figure 6 and
- a second example related to the training of a generative adversarial network device 800 illustrated by figure 8.

### AUTOENCODER NEURAL NETWORK

An autoencoder is a specific type of neural network with typically the same number of neurons in the input and the output. The output is expected and enforced to be as close as possible to the input during the training process. The aim of that 'copying machine' is based on the interesting feature that there is a bottleneck in one of the layers of the neural network that serves the purpose of compressing the information represented in the input. Such bottleneck layer represents what is called the latent space and every neural network layer (also called hidden layer) before that bottleneck layer aims at compressing the information of the input in the most performative way with a much smaller number of neurons than the input, such that the most prominent features of the initial input are represented in the latent space. This is also called the 'encoder' part of the autoencoder. Every hidden layer after the bottleneck layer aims at decompressing the information of the latent space such that the output is as similar possible to the input as possible. Such part of the autoencoder is called the 'decoder'.

The way the output is enforced to be as similar to the output as possible is through what is called the 'reconstruction loss' that compares quantity by quantity of the input (typically represented by a vector) to the outputs and sums up the differences. The aim of the learning process is to get the lowest possible number for that sum through a process called backpropagation.

Autoencoders are used in different tasks, such as in denoising images, also called denoising autoencoders. Denoising autoencoders, after learning the sparse representations of the latent space, can be presented with noisy images and are able to denoise them through the latent space learned, as they have learned to distinguish relevant features from noise.

Another kind of autoencoder that is specifically interesting to the current invention is variational autoencoders. Variational autoencoders not only learn the sparse representations of the latent space but are also able to generate new outputs as well.

What the autoencoders are learning is the data distribution of the inputs and what the variational autoencoders do is to split that problem into two subproblems: learn the median vector and the standard deviation vector of the latent space distribution, imposing that the latent space distribution is Gaussian (otherwise, we cannot ensure that a median or standard deviation exists). Once the training process is finished, a new generated output can be provided by sampling randomly from the distribution learned in the latent space and feeding it to the decoder, generating a completely new output that comes from a continuous representation of the data distribution of which the model was trained off. Such a sample is provided by a sampler designed to generate randomized and unique values.

To train the variational autoencoder, one needs to consider a final trick that is called the reparameterization trick. The sampling operation that is fed to the decoder cannot be trained through backpropagation, so in order to properly train the variational autoencoder one considers a deterministic mean and standard deviation, and the sampling processing is inserted in a multiplication of a random vector that has a Gaussian distribution with the deterministic standard deviation. Such a random vector does not go through the backpropagation process, but only the median and standard deviation value. Therefore, the sampling process is split through a deterministic part that is learned and a stochastic part that is fixed (and therefore not learned). Once the training is done, one can sample a random vector from a normal distribution and feed it to the decoder.

Variational autoencoders can be improved thanks to the quantization of the latent space that allows to employ what is called autoregressive models. The idea of such autoregressive models is that the generation is conditioned on its own previous values, therefore the first value of the output vector is a random value, the second value of the output is conditioned on the first value generated, the third value depends on the first and second value, etc. Such autoregressive models play the role of the decoder after the training process is finished.

An example architecture for a variational autoencoder training is a fully connected neural network comprising 1 to 5 hidden layers with each layer having a number of neurons equal to the previous layer's output. In particular embodiments, it is possible to apply non-linearity (any activation function like relu, prelu, elu, selu, swish, mish, sigmoid, tanh, tanhexp,) to some or all hidden layers and optionally to the output layer.

To train such an architecture, the following example may be implemented:
- as input, the encoder receives a concatenation of two separate character strings:
   - a first character string 605 representative of a composition or mixture (from 2 to 50 000 ingredients for example); an example of character strings 605: 'Woodleather', 'Lime Mint Berry', 'Lavender Haloscent', 'Melamine' and
   - a second character string 610 representative of associated hedonic, sensorial and/or physicochemical parameters such as defined above and/or optionally representative of non-hedonic, non-sensorial and/or non-physicochemical parameters such as price or a user preference indicator; an example of character strings 610: 'Soap Bar', 'Price: 10$', 'Musk, Vanilla, Spicy',' Carbon Renewable: 100%', 'Coconut',' Diary',' Vegetarian',' Natural:100%'
- the encoder 615 is configured to receive the input and comprises:
   - an input size (number of neurons) corresponding to the number of samples from the input,
   - an output size that can be configured depending on the level of compression desired,
   - a predictor of the mean of the latent space plugged to the output of the decoder and
   - a predictor of the standard deviation of the latent space. plugged to the output of the decoder,
- the latent space 620 is computed from the mean and standard deviation predicted using the reparameterization trick described as follows: the predicted standard deviation is multiplied by a random Gaussian noise and added to the predicted mean, the adjusted predicted mean and the predicted standard deviation are back propagated,
- the decoder 625:
   - is plugged to a concatenation of the computed latent space and to a second character string 630 representative of associated hedonic, sensorial and/or physicochemical parameter such as defined above and/or optionally representative of non-hedonic, non-sensorial and/or non-physicochemical parameters such as price or a user preference indicator, such a second character string 630 being identical to the first character string 610,
   - comprises an input size corresponding to the concatenation of the computed latent space and to each second character string representative and
   - comprises an output size equal to the input size of the encoder,
- during the backpropagation, the reconstruction loss and divergence loss are to be minimized. For reconstruction loss, this can be done using any metric capable of computing a distance (e.g., MSE, RMSE, KL-divergence...). For divergence loss, the objective is to enforce normality of the latent space using any metric capable of computing the distance between two distributions (e.g., KL-divergence, Wasserstein distance, etc.).

### GENERATIVE ADVERSARIAL NETWORK

Generative Adversarial Networks (GANs) are actually two neural networks: one generative model (G) and a discriminative model (D). The discriminative model learns the conditional probability of the target variable given the input variable. In the example 800 of figure 8, D learns to discriminate whether the composition that is fed into the neural network D is actually a sample of the real data or a generated sample. On the other hand, the neural network G learns the joint probability distribution of the input variable and the output variable. The generative model G is used to create new samples that should in principle follow the probability distribution of the real data, since G is learning the distribution function of the data itself.

These two models work in an adversarial setup. That means that they 'compete' with each other and eventually both of them gets better at their task. During the training process, a random vector is fed into the generative model G to produce an output typically called G(Z). Then, the output of the generator is passed to the discriminator. From time to time, a sample from the real data is fed into the discriminator. The discriminator outputs a single number: the probability its input belongs to the original data. The model D is maximizing its chances to predict the correct classes, but G, on the other hand, is trying to fool D. In the GAN literature, it is said that G and D are playing a two-player minimax game, meaning that one player is trying to maximize the probability of winning, while the other player is trying to minimize the probability of winning of the first player.

In this particular setup, some extra elements of complexity have been introduced, such as an extra discriminator that classifies whether a particular input from the generator or the real data belongs to a particular application (or conditioning medium), e.g. soap, shampoo, etc.

Once the training process reaches an endpoint, then one can use the generator to create new samples that are in principle representative of the materialized data. Indeed, the value function of the GAN model ensures that the probability distribution of the generated data is the probability distribution of the real data at the global minimum of the value function. Reaching the global minimum is the actual technical challenge that one must face when training GANs.

Different types of GANs exist and are of consideration for our present setup, such as infoGANs or Wasserstein GANs (WGAN). In the particular case of infoGANs, they are an information-theoretic extension of the GANs that are able to disentangle the latent space representations, such that you are able to see the most important features of the real data in an unsupervised manner. WGANs, on the other, improve the quality of the samples generated through the use of the Wasserstein distance that allows to compute the distance between different probability distributions (the real and generated probability distribution).

An example of such a training architecture is, for example, a fully connected neural network comprising:
- 1 to 5 hidden layers with each layer having a number of neurons equal to the previous layer's output. It is possible to apply non-linearity (any activation function like relu, prelu, elu, selu, swish, mish, sigmoid, tanh, tanhexp...) to some or all hidden layers and optionally to the output layer,
- a layer that computes the presence of the ingredients and applies the hadamard product between the presence and quantity and
- a random (e.g. normal) Gaussian vector that mimics a composition.

The corresponding training method may make use of the following parameters:
- as input:
   - a first character string 805 representative of a composition or mixture (from 2 to 50 000 ingredients for example) and
   - a second character string 810 representative of associated hedonic, sensorial and/or physicochemical parameters such as defined above and/or optionally representative of non-hedonic, non-sensorial and/or non-physicochemical parameters such as price or a user preference indicator,
- a target encoder plugged to the input corresponding to the second character strings 810 used to embed this input with output size varying from 100 to 1 000,
- a generator 815 plugged to the input with an output size that can vary from 100 to the 10 000 and plugged to the target encoder with output size varying from 100 to 1 000, said generator further making use of a concatenation of the input and target encoder values and an output size that can vary from 100 to the 10 000, said generator providing a composition with the number of possible ingredients that are used in the input,
- a real-fake discriminator 820, plugged randomly to either the overall input or to the generator, with output size equal to 1 as a classification for real or fake compositions,
- a discriminator target, plugged randomly to either the overall input or to the generator with output size equal to the number of targets as a classification for the desired targets and
- as backpropagation, the discriminator tries to maximize the critic loss, i.e. it tries to maximize the difference between its output on real instances and its output on generated instances and the generator tries to maximize the discriminator's output for its fake instances.

Figure 2 shows a particular embodiment of the method 200 object of the present invention. This computer-implemented method 200 for generating a fragrance or flavor composition represented by a digital identifier, comprises a step of generating 115 a fragrance or flavor composition using the trained autoencoder or generative adversarial network device trained according to any variant of the method disclosed above.

Such a step of generating 115 is performed, for example, by a computer program executed upon an electronic computing unit or computing system, such as a computer, for example. During this step, the trained machine learning device is executed to provide, as an output, a fragrance or flavor composition fitting predetermined values for the criteria (such as the hedonic, sensorial and/or physicochemical parameters mentioned above).

Below, two examples are presented:
- a first example related to the use of an autoencoder device 700 to generate fragrance or flavor compositions illustrated by figure 7 and
- a second example related to the training of a generative adversarial network device to generate fragrance or flavor compositions 900 illustrated by figure 9.

In the first example, a random (e.g. normal) Gaussian vector 705 that mimic the latent space during training is fed to a decoder 715. Ideally, this vector presents more than 100 dimensions to ensure the originality of the output by reducing the likeliness that the input has already been used by the decoder 715.

The input of the decoder 715 is a set of parameters such as price, olfactive or taste direction, application, stability, biodegradability, percent of renewable carbon, consumer liking, or other hedonic, sensorial and/or physicochemical parameter targets for the compositions that must match the set used during training as well as the concatenation of the latent space and target parameters.

The decoder 715, acting as a generator provides an output size equal to the number of ingredients used during training, that represents a composition or flavor or composition.

In the second example, a set of parameters such as price, olfactive or taste direction, application, stability, biodegradability, percent of renewable carbon, consumer liking, or other hedonic, sensorial and/or physicochemical parameter targets of acts as input targets for the compositions that must match the set used during training.

The target encoder is plugged to the input used to embed the parameter values with an output size varying from 100 to 1000.

The generator 915 uses a random vector 915 as well as targets 910 as an input to generate compositions matching said targets.

In particular embodiments, the step of generating 115 is configured to generate a fragrance or flavor composition digital identifier as a function of at least one value representative of at least one input constraint for generated compositions representative of at least one hedonic, sensorial and/or physicochemical parameter, for the generated fragrance or flavor composition digital identifier, among:
- an olfactive or taste direction associated to the identified fragrance or flavor composition,
- a conditioning medium associated to the identified fragrance or flavor composition,
- a visual and/or olfactive stability or degradation value associated to the identified fragrance or flavor composition,
- a percentage of biodegradability associated to the identified fragrance or flavor composition,
- a percentage of renewable carbon associated to the identified fragrance or flavor composition,
- a perceived psychophysical intensity associated to the identified fragrance or flavor composition,
- a flash point value associated to the identified fragrance or flavor composition,
- a toxicity value associated to the identified fragrance or flavor composition,
- a skin sensitization value associated to the identified fragrance or flavor composition,
- a number of ingredients in the composition,
- an enhancer compatibility value associated to the identified fragrance or flavor composition,
- an environmental accumulation value associated to the identified fragrance or flavor composition and.or
- retention indices for the composition.

In particular embodiments, the step 115 of generating is configured to generate a fragrance or flavor composition digital identifier associated to at least one secondary conditioning medium identifier as a function of an input of at least one fragrance or flavor composition digital identifier associated to a primary conditioning medium identifier.

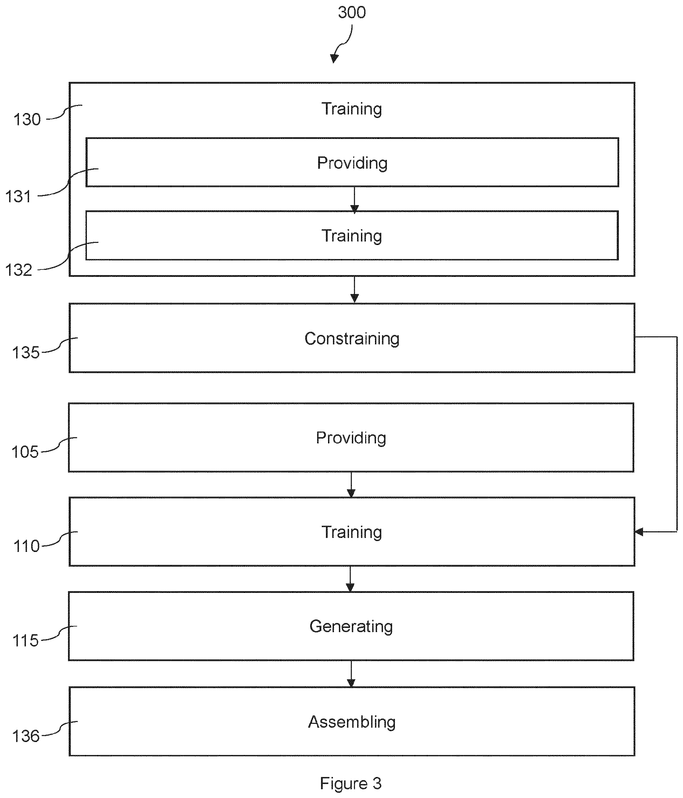

In particular embodiments, such as shown in figure 3, the computer-implemented method 300 according to any embodiment disclosed above, comprises:
- a step 130 of generating a trained auxiliary machine learning device, comprising:
   - providing 131 an original set of exemplar fragrance or flavor composition digital identifiers and labels representative of a chemical feature, such as olfaction or taste feature or character value (e.g. 'Musk',' Vanilla', 'Grilled Beef',' Strawberry',' Citrus'), a value representative of chemical compound quantity associated to said structure,
   - training 132 the auxiliary machine learning device using the original set of exemplar fragrance or flavor composition digital identifiers to provide a composition classifier and
- a step 135 of constraining a step of training a generative adversarial network device or an autoencoder device with the machine learning device, said step being executed during said step of training, as a reinforcement rule, or via postprocessing of the fragrance or flavor composition identifier generated.

The steps of generating 130, providing 131 and training 132 can be performed analogically to the method 100 and related steps of providing 105 and training 110 disclosed in regards of figure 1. Such a machine learning device may be a neural network device, for example.

The step of constraining 135 may be performed by using the output of the auxiliary machine learning device as a generation input for an autoencoder or generative adversarial network device. The output of such auxiliary machine learning device may be obtained, dynamically and after training, by inputting the generated composition into this auxiliary machine learning device and retroactively injecting the result into the autoencoder or generative adversarial network device.

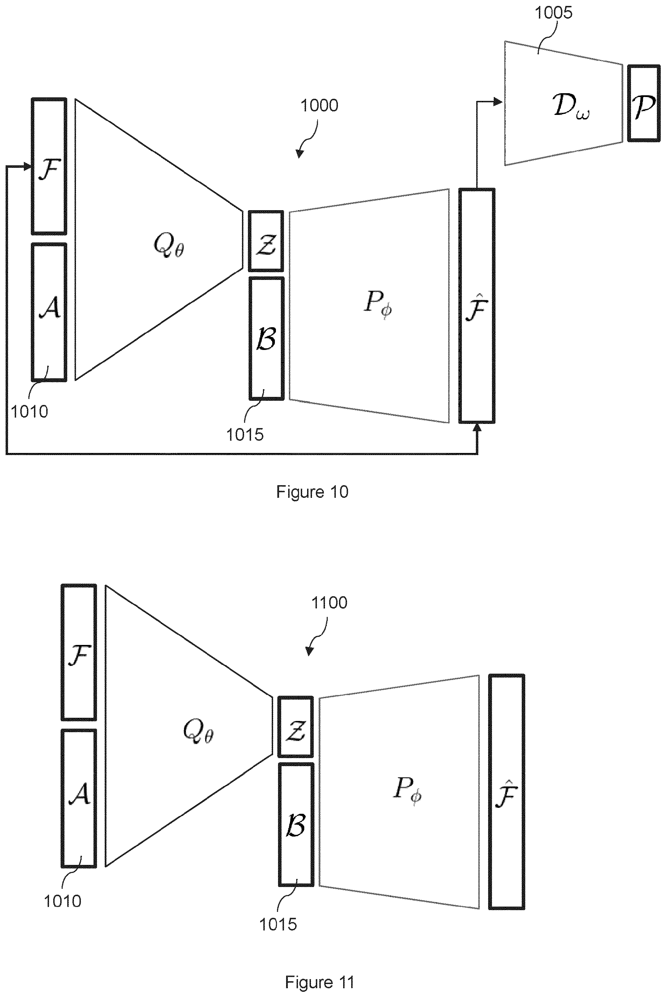

Such an auxiliary machine learning device 1005 is shown in figure 10 as a treatment of the generated data during by the training architecture 1000. The trained device 1100 is shown in figure 11. Such an auxiliary machine learning device 1005 is, for example, a discriminator neural network, such as one used in a GAN.

In further embodiments, the method may comprise a reinforcement mechanism in the training of the autoencoder device. Such a reinforcement mechanism may be, for example, a reinforcement loss to find new or desired solutions. Such a reinforcement rule is, for example, forcing the results to express a specific olfactive tonality.

**In** particular embodiments, the method, 100, 200 and/or 300, may comprise a step 136 of compounding a generated fragrance or flavor composition. Such a step 136 of compounding may be performed using any means known to one skilled in the art in order to obtain a fragrance or flavor composition. Such means may be, for example, means of synthetization in a laboratory or chemical plant.

**In** particular embodiments, such as shown in figure 1, the method 100 may comprise a step 137 of selecting a generated fragrance or flavor composition digital identifier to be compounded. Such a step 137 of compounding may be performed manually, via a user interface for example, or automatically, via the emission of compounding commands to a device capable of compounding the generated and selected chemical compound digital identifier.

As it is understood, the present invention aims at a computer-implemented autoencoder device trained according to any variant of the method 100 as shown in figure 1.

As it is understood, the present invention aims at a computer-implemented generative adversarial network device trained according to the method according to any variant of the method 100 as shown in figure 1.

As it is understood, the present invention aims at a computer program product comprising programming instructions to execute any variant of any method, 100, 200 or 300, as shown in figures 1 to 3.

As it is understood, the present invention aims at a computer-readable storage medium storing programming instruction that, when executed by a computer, imply that the computer executes the steps of any variant of any method, 100, 200 or 300, as shown in figures 1 to 3.

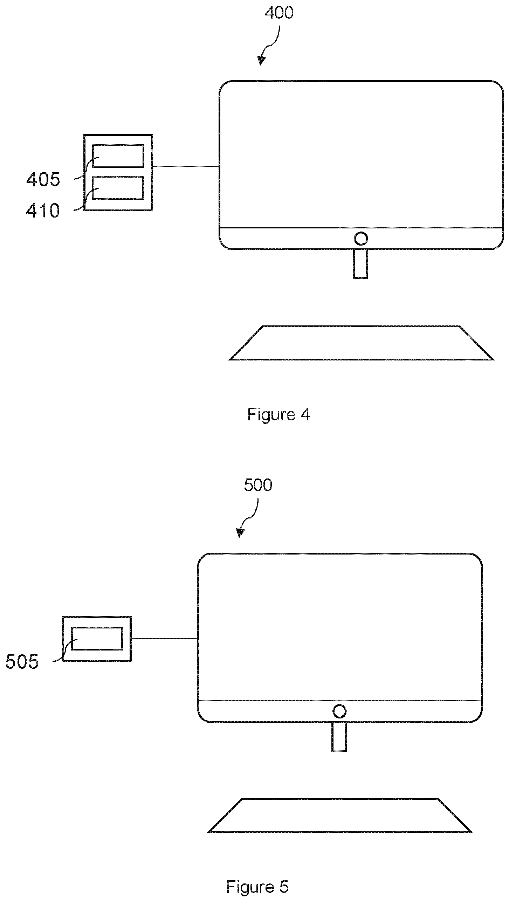

Figure 4 shows a particular embodiment of the device 400 object of the present invention. This device 400 for training an autoencoder neural network or generative adversarial network device to generate indeterministic and realistic digital representations of new fragrance or flavor ingredient compositions to be compounded, comprises the steps of:
- a means 405 of providing an original set of exemplar fragrance or flavor composition digital identifiers, said exemplar fragrance or flavor composition digital identifiers being representative of materialized fragrance or flavor compositions comprising at least two distinct ingredients and
- a means 410 of training an autoencoder device or generative adversarial network device using the original set of exemplar fragrance or flavor composition digital identifiers to generate a fragrance or flavor composition generative model trained to generate new fragrance or flavor ingredient compositions, comprising at least two distinct ingredients, to be compounded.

The means of providing 405 and training 410 correspond, mutatis mutandis, to the equivalent steps of providing 105 and training 110 of the method 100 object of the present invention.

Figure 5 shows a particular embodiment of the device 500 object of the present invention. This device 500 for generating a fragrance or flavor composition digital identifier, comprising a means of generating 505 a fragrance or flavor composition using the trained autoencoder or generative adversarial network device trained according to a particular embodiment of the method object of the present invention.

The means of generating 505 corresponds, mutatis mutandis, to the equivalent step of generating 115 of the method 100 object of the present invention.

Such use of autoencoders and generative adversarial neural networks may be used to generate targeted composition, defined by output criteria, and line extension. Line extension corresponds to the alteration of a composition to suit a different medium than the original medium for which the initial composition was designed.

Figures 10 and 11 show a particular embodiment of an autoencoder 1000 object of the present invention. In this embodiment, the input 1010 used for training representative of hedonic, sensorial and/or physicochemical parameters in the learning of the autoencoder is different than the input 1015 of the decoder such that the validity requirements for the generation stage differs from the initial requirements of the learning stage. For example, such a change might reflect, for example, a change in conditioning medium. Such embodiments allow the obtention of transformations in the compositions, for example, such as fitting a composition for a different conditioning medium than the conditioning medium of the exemplar dataset. Such features allow for line extensions, line extension referring to the change of perfumery or taste application.

Figures 10 and 11 also show, independently of the change in validity constraints, the use of an alternative set of compositions during the stage of generating. Such an alternative set may be random, rules-based or at least presenting a variation compared to the original dataset used in the training stage. Such an alternative set may correspond to the output of another generator, for example.

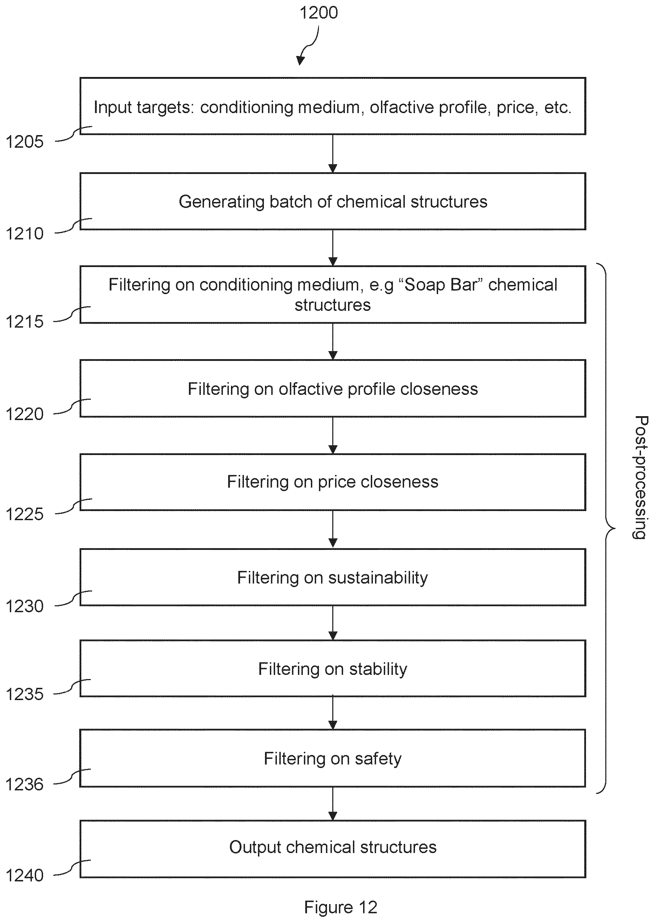

Figure 12 shows a particular embodiment of a post-processing method 1200 object of the present invention. This post-processing method 1200 comprises:
- a step 1205 of inputting generation targets for the generated compositions,
- a step 1210 of generating a batch of compositions,
- at least one of the following steps of post-processing:
   - a step 1215 of filtering on the conditioning medium identifier generated in the compositions,
   - a step 1225 of filtering on the price values of the compositions generated according to a range of validity,
   - a step 1230 of filtering on the sustainability value of the compositions generated according to a range of validity,
   - a step 1235 of filtering on the stability value of the compositions generated according to a range of validity and
   - a step 1236 of filtering on the safety value of the compositions generated according to a range of validity and
- a step 1240 of outputting the compositions that pass the post-processing step or steps.

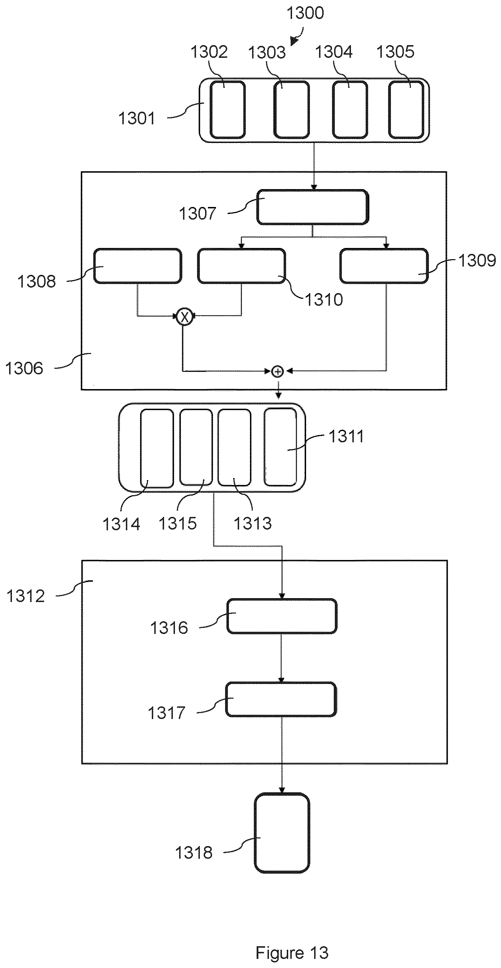

Figure 13 shows an example of a VAE training architecture 1300, in which:
- an input array 1301 of a size of 2321 items, comprising several types of input, including:
   - examplar compositions 1305, of an output size of 2000 items,
   - olfaction targets 1304, of an output size of 300 items,
   - application targets 1303, of an output size of 20 items and
   - a price target 1302, of an output size of 1 item,
- such an input array 1301 being fed to an encoder 1306, said encoder 1306 comprising:
   - a fully connected artificial neuron layer 1307, having an output size of 700 items,
   - a fully connected artificial neuron layer 1309 configured to be used as a mean of the output of the fully connected artificial neuron layer 1307, having an output size of 350,
   - a fully connected artificial neuron layer 1310 to be used as logarithmic variance of the output of the fully connected artificial neuron layer 1307, having an output size of 350,
   - a random Gaussian vector generator 1308, having an output size of 350, configured to be used in a product with the output of the processing layer 1310, the output of this product being concatenated with the output of the processing layer 1309
- the output of the encoder 1306 being used to generate a latent code 1311, having an output size of 350 items, which, when associated with other inputs for the decoder 1312, allow for the generation of new compositions, said other inputs including:
   - olfaction targets 1313, having an output size of 300 items,
   - application targets 1314, having an output size of 20 items and
   - a price target 1315, having an output size of 1 item,
- the decoder 1312 comprising:
   - two fully connected and cascading artificial neuron layers, 1316 and 1317, the first layer 1316 having an output size of 700 items and the second layer 1317 having an output size of 1000 items, providing a generated composition of 2000 ingredients or items.

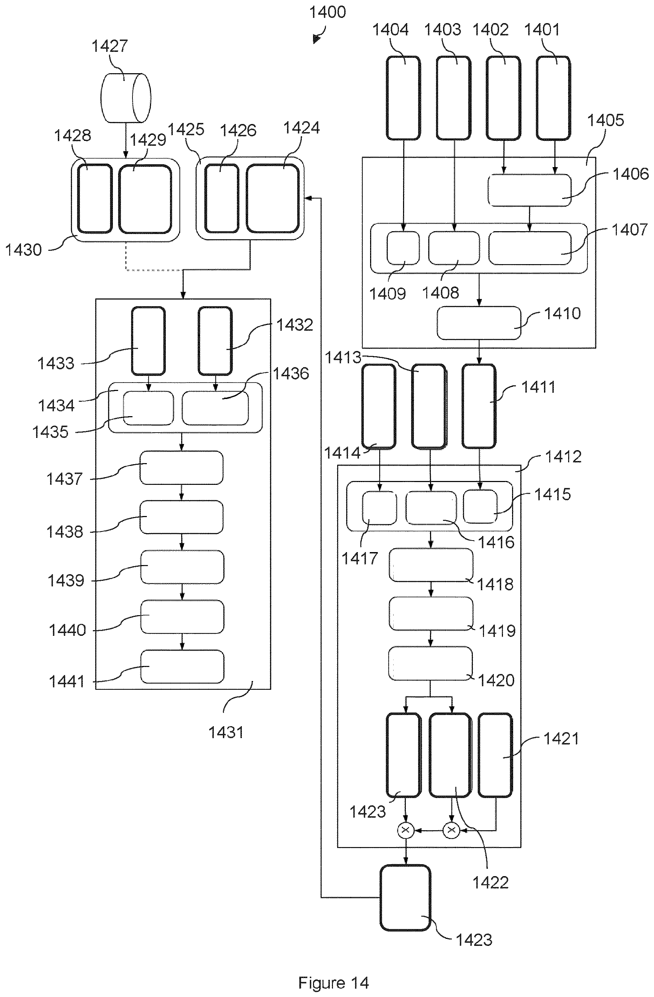

Figure 14 shows an example of a composition application extension training architecture 1400, configured to provide a composition output which is adapted to new applications, in which:
- an input array of a size of 2305 items, comprising several types of input, including:
   - examplar compositions 1401, having an output size of 2000 items,
   - examplar olfaction properties 1402 of said compositions, having an output size of 300 items,
   - examplar applications 1403 of said compositions, having an output size of 4 items and
   - a price target 1404, having an output size of 1 item,
- the input array being fed to an encoder 1405, said encoder 1405 comprising:
   - a fully connected artificial neuron layer 1406, having for input the exemplar compositions 1401 and the exemplar olfaction properties 1402 and having an output size of 512 items,
   - a fully connected artificial neuron layer 1407, having for input the output of the fully connected artificial neuron layer 1406 and having an output size of 512 items,
   - a fully connected artificial neuron layer 1408, having for input the examplar applications 1403 and having an output size of 256 items,
   - the price target 1404 acting as an input 1409 for a fully connected artificial neuron layer 1410, along with the output of the fully connected artificial neuron layers, 1407 and 1408,
   - the output of the fully connected artificial neuron layer 1410 having a size of 512 items and being used as a latent code 1411, said latent code 1411 acting as input 1415 in a decoder 1412 and having an output size of 512 items, along with an application target input 1413 and a price target input 1414,
- the decoder 1412 comprising:
   - a fully connected artificial neuron layer 1416, having for input the target application 1413 and having an output size of 256 items,
   - the price target 1417, along with the output of the fully connected artificial neuron layer 1416 and the latent code 1415 being fed to a series of three fully connected artificial neuron layers, 1418, 1419 and 1420, respectively having an output size of 512 items, 512 items and 1000 items,
   - the output of the fully connected artificial neuron layer 1420 is fed a redosing vector layer 1423, having an output of 2000 items, and into a replacement matrix layer 1422, having an output of 2000 x 2000 items, a product of the exemplar compositions 1421 with the replacement matrix 1422 being obtained and a product of this first product with the redosing vector 1423 being obtained to generate new compositions 1424,
- the generated new compositions 1424 are then used to constitute an alternative input 1425 for a discriminator 1431 comprising both:
   - the generated compositions 1424 and
   - the application target 1426 used in input for the decoder,
- another alternative input 1430, comprising real data for compounded compositions, can be fed into the discriminator 1431, said input 1430 comprising:
   - compounded compositions 1429 identifiers, extracted from a database 1427, having an output size of 2000, and
   - the application target 1428 for these compositions 1429, having an output size of 4,
- the discriminator 1431 comprising:
   - as an input:
      - compositions 1432, whether generated 1424 or real 1429, having an output size of 2000 ingredients,
      - target applications 1433, whether associated to the generated 1424 or real 1429 compositions, having an output size of 4,
   - a fully connected artificial neuron layer 1435 having, as an input, the target applications 1433, having an output size of 256,
   - the output of the fully connected artificial neuron layer 1435 being combined with the compositions 1432 and fed, as an input 1434 having a size of 2256 items, into a series of fully connected artificial neuron layers, 1437, 1438, 1439 and 1440, respectively having output sizes of 1024, 512, 512 and 1 items,
   - the output of the fully connected artificial neuron layer 1440 allowing for the discriminator 1431 to predict whether a composition 1432 is generated or real, that is having been compounded.

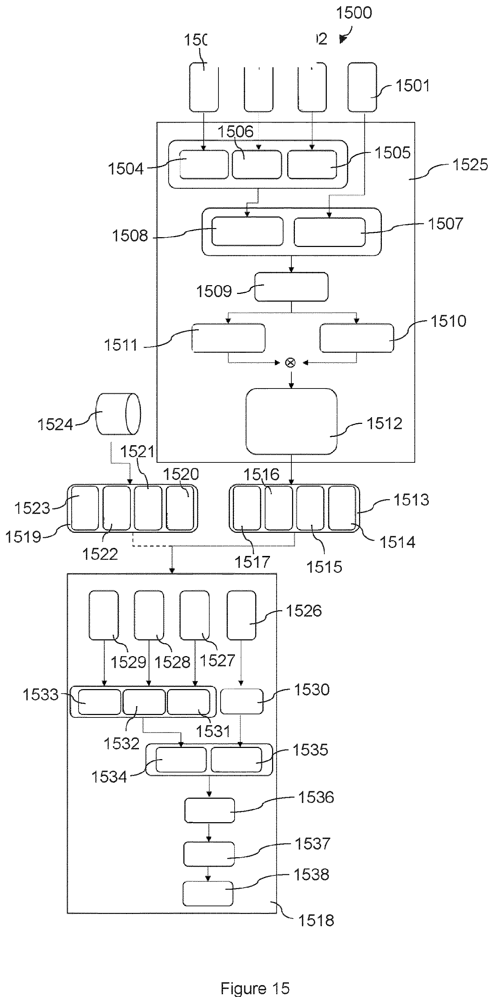

Figure 15 shows an example of a generative adversarial network training architecture 1500, configured to generate compositions, in which:
- an input comprising:
   - a noise vector 1501, having an output size of 100,
   - olfaction targets 1502, having an output size of 300,
   - application targets 1503, having an output size of 20,
   - a price target 1504, having an output size of 1,
- a generator 1525, comprising:
   - a fully connected artificial neuron layer 1505, having olfaction targets 1502 as an input, this layer 1505 having an output size of 30 items,
   - a fully connected artificial neuron layer 1506, having application targets 1503 as an input, this layer 1506 having an output size of 15 items,
   - the output of the fully connected artificial neuron layers, 1505, 1506, as well as the price target input 1504 being fed into a fully connected artificial neuron layer 1508, having an output size of 500 items,
   - the noise vector 1501 is fed into a fully connected artificial neuron layer 1507, having an output size of 500 items,
   - the output of the fully connected artificial neuron layers, 1507 and 1508, being fed into a fully connected artificial neuron layer 1509, having an output size of 1000 items,
   - the output of the fully connected artificial neuron layer 1509 is provided to two distinct fully connected artificial neuron layers, 1510 and 1511, both having an output size of 2000 items and providing, for layer 1510, ingredient identifiers to be present in the generated formula and, for layer 1511, quantity for said ingredients, the product of these outputs forming the generated composition 1512,
- the output generated composition 1514, along with the initially input olfaction target 1515, application target 1516 and price target 1517 form an alternative input 1513 for a discriminator 1518,
- an alternative input 1519, comprising:
   - real compositions 1520, having an output size of 2000 items, extracted from a real composition database 1524,
   - olfaction targets 1521, having an output size of 300 items,
   - application targets 1522, having an output size of 20 items, and
   - a price target 1523 having an output size of 1 item,
- the input 1513 and the alternative input 1519 being fed into the discriminator 1518,
- the discriminator 1518 comprising:
   - an input comprising:
      - composition 1526 identifiers, originating either from the input 1513 or from the alternative input 1519, having an output size of 2000 items,
      - olfaction targets 1527, originating either from the input 1513 or from the alternative input 1519, having an output size of 300 items,
      - application targets 1528, originating either from the input 1513 or from the alternative input 1519, having an output size of 20 items and
      - a price target 1529, originating either from the input 1513 or from the alternative input 1519, having an output size of 1 item,
   - a fully connected artificial neuron layer 1530, using as input the composition 1526 identifiers and having an output size of 30 items,
   - a fully connected artificial neuron layer 1531, using as input the olfaction targets 1527 and having an output size of 30 items,
   - a fully connected artificial neuron layer 1532, using as input the application targets 1528 and having an output size of 15 items,
   - the price target 1533, along with the output of the fully connected artificial neuron layers, 1531 and 1532, being fed into a fully connected artificial neuron layer 1534, having an output size of 500 items,
   - a fully connected artificial neuron layer 1535, having the output of the fully connected artificial neuron layer 1530 as an input, and having an output size of 500,
   - a fully connected artificial neuron layer 1536, having the output of the fully connected artificial neuron layers, 1535 and 1534, as an input, and having an output size of 100,
   - a fully connected artificial neuron layer 1537, having the output of the fully connected artificial neuron layer 1536 as an input, and having an output size of 1,
   - the output of the fully connected artificial neuron layer 1537 providing a prediction 1538 as to whether a composition identifier 1526 is real or generated.

## Claims

1. Computer-implemented method (100) for training an autoencoder neural network or generative adversarial network device to generate indeterministic and realistic digital representations of new fragrance or flavor ingredient compositions to be compounded, **characterized in that** it comprises the steps of:
- providing (105) an original set of exemplar fragrance or flavor composition digital identifiers, said exemplar fragrance or flavor composition digital identifiers being representative of materialized fragrance or flavor compositions comprising at least two distinct fragrance or flavor compound ingredients, the original set of exemplar fragrance or flavor composition digital identifier further comprising, associated to at least one exemplar fragrance or flavor composition digital identifier, a value representative of at least one hedonic, sensorial and/or physicochemical parameter, and
- training (110) an autoencoder device or generative adversarial network device using the original set of exemplar fragrance or flavor composition digital identifiers to generate a fragrance or flavor composition generative model trained to generate new fragrance or flavor ingredient compositions, comprising at least two distinct fragrance or flavor compound ingredients, to be compounded, as a function of at least one value representative of at least one constraint for generated compositions representative of at least one hedonic, sensorial and/or physicochemical parameter, for the new generated fragrance or flavor composition.

2. Computer-implemented method (100) according to claim 1, in which the value representative of at least one hedonic, sensorial and/or physicochemical parameter, said value being representative of at least one captured hedonic, sensorial and/or physicochemical parameter for the materialized fragrance or flavor composition, is selected from among:
- an olfactive or taste direction associated to the identified fragrance or flavor composition,
- a conditioning medium associated to the identified fragrance or flavor composition,
- a visual and/or olfactive stability or degradation value associated to the identified fragrance or flavor composition,
- a percentage of biodegradability associated to the identified fragrance or flavor composition,
- a percentage of renewable carbon associated to the identified fragrance or flavor composition,
- a perceived psychophysical intensity associated to the identified fragrance or flavor composition,
- a flash point value associated to the identified fragrance or flavor composition,
- a toxicity value associated to the identified fragrance or flavor composition,
- a skin sensitization value associated to the identified fragrance or flavor composition,
- an enhancer compatibility value associated to the identified fragrance or flavor composition,
- a number of ingredients in the composition,
- an environmental accumulation value associated to the identified fragrance or flavor composition and/or
- retention indices for the composition

3. Method (100) according to claim 2, which comprises a step (104) of capturing a value representative of at least one hedonic, sensorial and/or physicochemical parameter for a least one materialized fragrance or flavor composition, said value being associated to a digital identifier of a fragrance or flavor composition in the exemplar set.

4. Computer-implemented method (100) according to any one of claims 1 to 3, in which the step (110) of training is configured to train a variational autoencoder device.

5. Computer-implemented method (100) according to any one of claims 1 to 4, in which:
- the step (105) of providing is configured to provide an original set of exemplar fragrance or flavor composition digital identifiers associated to a primary conditioning medium identifier and at least an additional original set of exemplar fragrance or flavor composition digital identifiers associated to at least one secondary conditioning medium identifier and
- the step (110) of training is configured to train a generative model in which the input is a fragrance or flavor composition digital identifier associated to a primary conditioning medium and the output is a fragrance or flavor composition digital identifier associated to at least one secondary conditioning medium identifier.

6. Computer-implemented method (200) for generating a fragrance or flavor composition represented by a digital identifier, **characterized in that** it comprises a step of generating (115) a fragrance or flavor composition using the trained autoencoder or generative adversarial network device trained according to any one of claims 1 to 5.

7. Computer-implemented method (200) according to claim 6, in which the step of generating (115) is configured to generate a fragrance or flavor composition digital identifier as a function of at least one value representative of at least one input constraint for generated compositions representative of at least one hedonic, sensorial and/or physicochemical parameter, for the generated fragrance or flavor composition digital identifier, among:
- an olfactive or taste direction associated to the identified fragrance or flavor composition,
- a conditioning medium associated to the identified fragrance or flavor composition,
- a visual and/or olfactive stability or degradation value associated to the identified fragrance or flavor composition,
- a percentage of biodegradability associated to the identified fragrance or flavor composition,
- a percentage of renewable carbon associated to the identified fragrance or flavor composition,
- a perceived psychophysical intensity associated to the identified fragrance or flavor composition,
- a flash point value associated to the identified fragrance or flavor composition,
- a toxicity value associated to the identified fragrance or flavor composition,
- a skin sensitization value associated to the identified fragrance or flavor composition,
- an enhancer compatibility value associated to the identified fragrance or flavor composition
- a number of ingredients in the composition,
- an environmental accumulation value associated to the identified fragrance or flavor composition and/or
- retention indices for the composition.

8. Computer-implemented method (200) according to claim 5 and any one of claims 6 or 7, in which the step (115) of generating is configured to generate a fragrance or flavor composition digital identifier associated to at least one secondary conditioning medium identifier as a function of an input of at least one fragrance or flavor composition digital identifier associated to a primary conditioning medium identifier.

9. Computer-implemented method (300) according to any one of claims 1 to 7, which comprises:
- a step (130) of generating a trained auxiliary machine learning device, comprising:
- providing (131) an original set of exemplar fragrance or flavor composition digital identifiers and labels representative of a chemical feature, such as an olfaction feature value or a value representative of chemical compound quantity associated to said structure,
- training (132) the auxiliary machine learning device using the original set of exemplar fragrance or flavor composition digital identifiers to provide a composition classifier and
- a step (135) of constraining the step of training a generative adversarial network device or an autoencoder device with the machine learning device, said step being executed during said step of training, as a reinforcement rule, or via postprocessing of the fragrance or flavor composition identifier generated.

10. Method (100, 200, 300) according to any one of claims 1 to 9, which further comprises a step (136) of compounding a generated fragrance or flavor composition comprising at least two distinct ingredients.

11. Method (100, 200, 300) according to any one of claims 1 to 10, which further comprises a step (137) of selecting a generated fragrance or flavor composition, comprising at least two distinct ingredients, to be compounded.

12. Computer-implemented autoencoder device trained according to the method according to any one of claims 1 to 5.

13. Computer-implemented generative adversarial network device trained according to the method according to any one of claims 1 to 5.

14. Computer program product comprising instructions which, when the program is executed by a computer, cause the computer to carry out the method according to any one of claims 1 to 11.

15. Computer-readable storage medium storing instructions which, when executed by a computer, cause the computer to carry out the steps of any one of claims 1 to 11.

16. Device (400) for training an autoencoder neural network or generative adversarial network device to generate indeterministic and realistic digital representations of new fragrance or flavor ingredient compositions to be compounded, **characterized in that** it comprises the steps of:
- a means (405) of providing an original set of exemplar fragrance or flavor composition digital identifiers, said exemplar fragrance or flavor composition digital identifiers being representative of materialized fragrance or flavor compositions comprising at least two distinct fragrance or flavor compound ingredients, the original set of exemplar fragrance or flavor composition digital identifier further comprising, associated to at least one exemplar fragrance or flavor composition digital identifier, a value representative of at least one hedonic, sensorial and/or physicochemical parameter, and
- a means (410) of training an autoencoder device or generative adversarial network device using the original set of exemplar fragrance or flavor composition digital identifiers to generate a fragrance or flavor composition generative model trained to generate new fragrance or flavor ingredient compositions, comprising at least two distinct fragrance or flavor compound ingredients, to be compounded, as a function of at least one value representative of at least one constraint for generated compositions representative of at least one hedonic, sensorial and/or physicochemical parameter, for the new generated fragrance or flavor composition.

17. Device (500) for generating a fragrance or flavor composition digital identifier, **characterized in that** it comprises a means of generating (505) a fragrance or flavor composition, comprising at least two distinct ingredients, using the trained autoencoder or generative adversarial network device trained according to any one of claim 1 to 5.

## Patentansprüche

1. Rechnerimplementiertes Verfahren (100) zum Trainieren einer Autocodierer-Neuronalnetz- oder generativen Gegnernetzvorrichtung, um indeterministische und realistische digitale Repräsentationen von neuen Duftstoff- oder Geschmacksstoff-Bestandteilszusammensetzungen, die zusammenzustellen sind, zu erzeugen, **dadurch gekennzeichnet, dass** es die folgenden Schritte umfasst:
- Bereitstellen (105) eines ursprünglichen Satzes von beispielhaften digitalen Kennungen von Duftstoff- oder Geschmacksstoffzusammensetzungen, wobei die beispielhaften digitalen Kennungen von Duftstoff- oder Geschmacksstoffzusammensetzungen repräsentativ für materialisierte Duftstoff- oder Geschmacksstoffzusammensetzungen sind, die mindestens zwei unterschiedliche Duftstoff- oder Geschmacksstoffverbindungsbestandteile umfassen, wobei der ursprüngliche Satz von beispielhaften digitalen Kennungen von Duftstoff- oder Geschmacksstoffzusammensetzungen ferner, verknüpft mit mindestens einer beispielhaften digitalen Kennung von Duftstoff- oder Geschmacksstoffzusammensetzungen, einen Wert umfasst, der repräsentativ für mindestens einen hedonischen, sensorischen und/oder physiochemischen Parameter ist, und
- Trainieren (110) einer Autocodierervorrichtung oder generativen Gegnernetzvorrichtung unter Verwendung des ursprünglichen Satzes von beispielhaften digitalen Kennungen von Duftstoff- oder Geschmacksstoffzusammensetzungen, um ein generatives Duftstoff- oder Geschmacksstoffzusammensetzungsmodell zu erzeugen, trainiert, um neue Duftstoff- oder Geschmacksstoffzusammensetzungen, die mindestens zwei unterschiedliche Duftstoff- oder Geschmacksstoffverbindungsbestandteile umfassen, die zusammenzustellen sind, zu erzeugen, in Abhängigkeit von mindestens einem Wert, der repräsentativ für mindestens eine Einschränkung für erzeugte Zusammensetzungen ist, die repräsentativ für mindestens einen hedonischen, sensorischen und/oder physiochemischen Parameter für die neu erzeugte Duftstoff- oder Geschmacksstoffzusammensetzung ist.

2. Rechnerimplementiertes Verfahren (100) nach Anspruch 1, bei dem der Wert, der repräsentativ für mindestens einen hedonischen, sensorischen und/oder physiochemischen Parameter ist, wobei der Wert repräsentativ für mindestens einen hedonischen, sensorischen und/oder physiochemischen Parameter für die materialisierte Duftstoff- oder Geschmacksstoffzusammensetzung ist, ausgewählt wird unter Folgendem:
- einer mit der identifizierten Duftstoff- oder Geschmacksstoffzusammensetzung verknüpften olfaktorischen oder geschmacklichen Richtung,
- einem mit der identifizierten Duftstoff- oder Geschmacksstoffzusammensetzung verknüpften Aufbereitungsmedium,
- einem mit der identifizierten Duftstoff- oder Geschmacksstoffzusammensetzung verknüpften Wert visueller und/oder olfaktorischer Stabilität oder Verschlechterung,
- einem mit der identifizierten Duftstoff- oder Geschmacksstoffzusammensetzung verknüpften Prozentsatz biologischer Abbaubarkeit,
- einem mit der identifizierten Duftstoff- oder Geschmacksstoffzusammensetzung verknüpften Prozentsatz von erneuerbarem Kohlenstoff,
- einer mit der identifizierten Duftstoff- oder Geschmacksstoffzusammensetzung verknüpften wahrgenommenen psychophysischen Intensität,
- einem mit der identifizierten Duftstoff- oder Geschmacksstoffzusammensetzung verknüpften Flammpunktwert,
- einem mit der identifizierten Duftstoff- oder Geschmacksstoffzusammensetzung verknüpften Toxizitätswert,
- einem mit der identifizierten Duftstoff- oder Geschmacksstoffzusammensetzung verknüpften Hautsensibilisierungswert,
- einem mit der identifizierten Duftstoff- oder Geschmacksstoffzusammensetzung verknüpften Verstärkerverträglichkeitswert,
- einer Anzahl von Bestandteilen in der Zusammensetzung,
- einem mit der identifizierten Duftstoff- oder Geschmacksstoffzusammensetzung verknüpften Umweltakkumulationswert und/oder
- Retentionsindices für die Zusammensetzung.

3. Verfahren (100) nach Anspruch 2, das einen Schritt (104) des Erfassens eines Werts umfasst, der repräsentativ für mindestens einen hedonischen, sensorischen und/oder physiochemischen Parameter für mindestens eine materialisierte Duftstoff- oder Geschmacksstoffzusammensetzung ist, wobei der Wert mit einer digitalen Kennung einer Duftstoff- oder Geschmacksstoffzusammensetzung in dem beispielhaften Satz verknüpft ist.

4. Rechnerimplementiertes Verfahren (100) nach einem der Ansprüche 1 bis 3, wobei der Schritt (110) des Trainierens dafür konfiguriert ist, eine Variationsautocodierervorrichtung zu trainieren.

5. Rechnerimplementiertes Verfahren (100) nach einem der Ansprüche 1 bis 4, bei dem:
- der Schritt (105) des Bereitstellens dafür konfiguriert ist, einen mit einer Kennung eines primären Aufbereitungsmediums verknüpften ursprünglichen Satz von beispielhaften digitalen Kennungen von Duftstoff- oder Geschmacksstoffzusammensetzungen und mindestens einen mit mindestens einer Kennung eines sekundären Aufbereitungsmediums verknüpften zusätzlichen ursprünglichen Satz von beispielhaften digitalen Kennungen von Duftstoff- oder Geschmacksstoffzusammensetzungen bereitzustellen, und
- der Schritt (110) des Trainierens dafür konfiguriert ist, ein generatives Modell zu trainieren, in dem die Eingabe eine mit einem primären Aufbereitungsmedium verknüpfte digitale Kennung einer Duftstoff- oder Geschmacksstoffzusammensetzung ist und die Ausgabe eine mit mindestens einer Kennung eines sekundären Aufbereitungsmediums verknüpfte digitale Kennung einer Duftstoff- oder Geschmacksstoffzusammensetzung ist.

6. Rechnerimplementiertes Verfahren (200) zum Erzeugen einer durch eine digitale Kennung repräsentierten Duftstoff- oder Geschmacksstoffzusammensetzung, **dadurch gekennzeichnet, dass** es einen Schritt des Erzeugens (115) einer Duftstoff- oder Geschmacksstoffzusammensetzung unter Verwendung der nach einem der Ansprüche 1 bis 5 trainierten Autocodierer- oder generativen Gegnernetzvorrichtung umfasst.

7. Rechnerimplementiertes Verfahren (200) nach Anspruch 6, bei dem der Schritt des Erzeugens (115) dafür konfiguriert ist, eine digitale Kennung einer Duftstoff- oder Geschmacksstoffzusammensetzung in Abhängigkeit von mindestens einem Wert zu erzeugen, der repräsentativ für mindestens eine Eingabeeinschränkung für erzeugte Zusammensetzungen ist, die repräsentativ für mindestens einen hedonischen, sensorischen und/oder physiochemischen Parameter für die erzeugte Duftstoff- oder Geschmacksstoffzusammensetzung ist, unter:
- einer mit der identifizierten Duftstoff- oder Geschmacksstoffzusammensetzung verknüpften olfaktorischen oder geschmacklichen Richtung,
- einem mit der identifizierten Duftstoff- oder Geschmacksstoffzusammensetzung verknüpften Aufbereitungsmedium,
- einem mit der identifizierten Duftstoff- oder Geschmacksstoffzusammensetzung verknüpften Wert visueller und/oder olfaktorischer Stabilität oder Verschlechterung,
- einem mit der identifizierten Duftstoff- oder Geschmacksstoffzusammensetzung verknüpften Prozentsatz biologischer Abbaubarkeit,
- einem mit der identifizierten Duftstoff- oder Geschmacksstoffzusammensetzung verknüpften Prozentsatz von erneuerbarem Kohlenstoff,
- einer mit der identifizierten Duftstoff- oder Geschmacksstoffzusammensetzung verknüpften wahrgenommenen psychophysischen Intensität,
- einem mit der identifizierten Duftstoff- oder Geschmacksstoffzusammensetzung verknüpften Flammpunktwert,
- einem mit der identifizierten Duftstoff- oder Geschmacksstoffzusammensetzung verknüpften Toxizitätswert,
- einem mit der identifizierten Duftstoff- oder Geschmacksstoffzusammensetzung verknüpften Hautsensibilisierungswert,
- einem mit der identifizierten Duftstoff- oder Geschmacksstoffzusammensetzung verknüpften Verstärkerverträglichkeitswert,
- einer Anzahl von Bestandteilen in der Zusammensetzung,
- einem mit der identifizierten Duftstoff- oder Geschmacksstoffzusammensetzung verknüpften Umweltakkumulationswert und/oder
- Retentionsindices für die Zusammensetzung.

8. Rechnerimplementiertes Verfahren (200) nach Anspruch 5 und einem der Ansprüche 6 oder 7, bei dem der Schritt des Erzeugens (115) dafür konfiguriert ist, eine mit mindestens einer Kennung eines sekundären Aufbereitungsmediums verknüpfte digitale Kennung einer Duftstoff- oder Geschmacksstoffzusammensetzung in Abhängigkeit von einer Eingabe mindestens einer mit einer Kennung eines primären Aufbereitungsmediums verknüpften digitalen Kennung einer Duftstoff- oder Geschmacksstoffzusammensetzung zu erzeugen.

9. Rechnerimplementiertes Verfahren (300) nach einem der Ansprüche 1 bis 7, das Folgendes umfasst:
- einen Schritt (130) des Erzeugens einer trainierten unterstützenden Maschinenlernvorrichtung, der Folgendes umfasst:
- Bereitstellen (131) eines ursprünglichen Satzes von beispielhaften digitalen Kennungen von Duftstoff- oder Geschmacksstoffzusammensetzungen und Markierungen, die repräsentativ sind für ein chemisches Merkmal, wie beispielsweise einen olfaktorischen Merkmalswert oder einen Wert, der für eine mit der Struktur verknüpfte Menge einer chemischen Verbindung ist,
- Trainieren (132) der unterstützenden Maschinenlernvorrichtung unter Verwendung des ursprünglichen Satzes von beispielhaften digitalen Kennungen von Duftstoff- oder Geschmacksstoffzusammensetzungen, um einen Zusammensetzungsklassifikator bereitzustellen, und
- einen Schritt (135) des Einschränkens des Schritts des Trainierens einer generativen Gegnernetzvorrichtung oder einer Autocodierervorrichtung mit der Maschinenlernvorrichtung, wobei der Schritt während des Schritts des Trainierens, als eine Verstärkungsregel oder über Nachbearbeiten der erzeugten Kennung von Duftstoff- oder Geschmacksstoff-Zusammensetzungen ausgeführt wird.

10. Verfahren (100, 200, 300) nach einem der Ansprüche 1 bis 9, das ferner einen Schritt (136) des Zusammenstellens einer mindestens zwei unterschiedliche Bestandteile umfassenden erzeugten Duftstoff- oder Geschmacksstoffzusammensetzung umfasst.

11. Verfahren (100, 200, 300) nach einem der Ansprüche 1 bis 10, das ferner einen Schritt (137) des Auswählens einer mindestens zwei unterschiedliche Bestandteile umfassenden erzeugten Duftstoff- oder Geschmacksstoffzusammensetzung, die zusammenzustellen ist, umfasst.

12. Rechnerimplementierte Autocodierervorrichtung, trainiert gemäß dem Verfahren nach einem der Ansprüche 1 bis 5.

13. Rechnerimplementierte generative Gegnernetzvorrichtung, trainiert gemäß dem Verfahren nach einem der Ansprüche 1 bis 5.

14. Rechnerprogrammerzeugnis, das Anweisungen umfasst, die, wenn das Programm durch einen Rechner ausgeführt wird, veranlassen, dass der Rechner das Verfahren nach einem der Ansprüche 1 bis 11 vollzieht.

15. Rechnerlesbares Speichermedium, das Anweisungen speichert, die, wenn sie durch einen Rechner ausgeführt werden, veranlassen, dass der Rechner die Schritte nach einem der Ansprüche 1 bis 11 vollzieht.

16. Vorrichtung (400) zum Trainieren einer Autocodierer-Neuronalnetz- oder generativen Gegnernetzvorrichtung, um indeterministische und realistische digitale Repräsentationen von neuen Duftstoff- oder Geschmacksstoff-Bestandteilszusammensetzungen, die zusammenzustellen sind, zu erzeugen, **dadurch gekennzeichnet, dass** sie Folgendes umfasst:
- ein Mittel (405) zum Bereitstellen eines ursprünglichen Satzes von beispielhaften digitalen Kennungen von Duftstoff- oder Geschmacksstoffzusammensetzungen, wobei die beispielhaften digitalen Kennungen von Duftstoff- oder Geschmacksstoffzusammensetzungen repräsentativ für materialisierte Duftstoff- oder Geschmacksstoffzusammensetzungen sind, die mindestens zwei unterschiedliche Duftstoff- oder Geschmacksstoffverbindungsbestandteile umfassen, wobei der ursprüngliche Satz von beispielhaften digitalen Kennungen von Duftstoff- oder Geschmacksstoffzusammensetzungen ferner, verknüpft mit mindestens einer beispielhaften digitalen Kennung von Duftstoff- oder Geschmacksstoffzusammensetzungen, einen Wert umfasst, der repräsentativ für mindestens einen hedonischen, sensorischen und/oder physiochemischen Parameter ist, und
- ein Mittel (410) zum Trainieren einer Autocodierervorrichtung oder generativen Gegnernetzvorrichtung unter Verwendung des ursprünglichen Satzes von beispielhaften digitalen Kennungen von Duftstoff- oder Geschmacksstoffzusammensetzungen, um ein generatives Duftstoff- oder Geschmacksstoffzusammensetzungsmodell zu erzeugen, trainiert, um neue Duftstoff- oder Geschmacksstoff-Bestandteilszusammensetzungen, die mindestens zwei unterschiedliche Duftstoff- oder Geschmacksstoffverbindungsbestandteile umfassen, die zusammenzustellen sind, zu erzeugen, in Abhängigkeit von mindestens einem Wert, der repräsentativ für mindestens eine Einschränkung für erzeugte Zusammensetzungen ist, die repräsentativ für mindestens einen hedonischen, sensorischen und/oder physiochemischen Parameter für die neu erzeugte Duftstoff- oder Geschmacksstoffzusammensetzung ist.

17. Vorrichtung (500) zum Erzeugen einer digitalen Kennung einer Duftstoff- oder Geschmacksstoffzusammensetzung, **dadurch gekennzeichnet, dass** sie ein Mittel zum Erzeugen (505) einer Duftstoff- oder Geschmacksstoffzusammensetzung, die mindestens zwei unterschiedliche Bestandteile umfasst, unter Verwendung der trainierten Autocodierer- oder generativen Gegnernetzvorrichtung, trainiert nach einem der Ansprüche 1 bis 5, umfasst.

## Revendications

1. Procédé mis en œuvre sur ordinateur (100) pour l'entraînement d'un dispositif de réseau neuronal auto-encodeur ou de réseau antagoniste génératif pour générer des représentations numériques indéterministes et réalistes de nouvelles compositions d'ingrédients parfumés ou aromatisés à composer, **caractérisé en ce qu'**il comprend les étapes :
- de fourniture (105) d'un ensemble original d'identifiants numériques de compositions parfumées ou aromatisées à titre d'exemples, lesdits identifiants numériques de compositions parfumées ou aromatisées à titre d'exemples étant représentatifs de compositions parfumées ou aromatisées matérialisées comprenant au moins deux ingrédients composés parfumés ou aromatisés distincts, l'ensemble original d'identifiants numériques de compositions parfumées ou aromatisées à titre d'exemples comprenant en outre, associée à au moins un identifiant numérique de composition parfumée ou aromatisée à titre d'exemple, une valeur représentative d'au moins un paramètre hédonique, sensoriel et/ou physico-chimique, et
- d'entraînement (110) d'un dispositif auto-encodeur ou dispositif de réseau antagoniste génératif en utilisant l'ensemble original d'identifiants numériques de compositions parfumées ou aromatisées à titre d'exemples pour générer un modèle génératif de compositions parfumées ou aromatisées entraîné pour générer des nouvelles compositions d'ingrédients parfumés ou aromatisés, comprenant au moins deux ingrédients composés parfumés ou aromatisés distincts, à composer, en fonction d'au moins une valeur représentative d'au moins une contrainte pour des compositions générées représentatives d'au moins un paramètre hédonique, sensoriel et/ou physico-chimique, pour la nouvelle composition parfumée ou aromatisée générée.

2. Procédé mis en œuvre sur ordinateur (100) selon la revendication 1, dans lequel la valeur représentative d'au moins un paramètre hédonique, sensoriel et/ou physico-chimique, ladite valeur étant représentative d'au moins un paramètre hédonique, sensoriel et/ou physico-chimique capturé pour la composition parfumée ou aromatisée matérialisée, est choisie parmi :
- une direction olfactive ou gustative associée à la composition parfumée ou aromatisée identifiée,
- une substance de conditionnement associée à la composition parfumée ou aromatisée identifiée,
- une valeur de stabilité ou de dégradation visuelle et/ou olfactive associée à la composition parfumée ou aromatisée identifiée,
- un pourcentage de biodégradabilité associé à la composition parfumée ou aromatisée identifiée,
- un pourcentage de carbone renouvelable associé à la composition parfumée ou aromatisée identifiée,
- une intensité psychophysique perçue associée à la composition parfumée ou aromatisée identifiée,
- une valeur de point d'éclair associée à la composition parfumée ou aromatisée identifiée,
- une valeur de toxicité associée à la composition parfumée ou aromatisée identifiée,
- une valeur de sensibilisation cutanée associée à la composition parfumée ou aromatisée identifiée,
- une valeur de compatibilité d'exhausteur associée à la composition parfumée ou aromatisée identifiée,
- un nombre d'ingrédients dans la composition,
- une valeur d'accumulation environnementale associée à la composition parfumée ou aromatisée identifiée et/ou
- des indices de rétention pour la composition.

3. Procédé (100) selon la revendication 2, qui comprend une étape (104) de saisie d'une valeur représentative d'au moins un paramètre hédonique, sensoriel et/ou physico-chimique pour au moins une composition parfumée ou aromatisée matérialisée, ladite valeur étant associée à un identifiant numérique d'une composition parfumée ou aromatisée dans l'ensemble à titre d'exemple.

4. Procédé mis en œuvre sur ordinateur (100) selon l'une quelconque des revendications 1 à 3, dans lequel l'étape (110) d'entraînement est configurée pour entraîner un dispositif auto-encodeur variationnel.

5. Procédé mis en œuvre sur ordinateur (100) selon l'une quelconque des revendications 1 à 4, dans lequel :
- l'étape (105) de fourniture est configurée pour fournir un ensemble original d'identifiants numériques de compositions parfumées ou aromatisées à titre d'exemples associés à un identifiant de substance de conditionnement primaire et au moins un ensemble original supplémentaire d'identifiants numériques de compositions parfumées ou aromatisées à titre d'exemples associés à au moins un identifiant de substance de conditionnement secondaire et
- l'étape (110) d'entraînement est configurée pour entraîner un modèle génératif dans lequel l'entrée est un identifiant numérique de composition parfumée ou aromatisée associé à une substance de conditionnement primaire et la sortie est un identifiant numérique de composition parfumée ou aromatisée associé à au moins un identifiant de substance de conditionnement secondaire.

6. Procédé mis en œuvre sur ordinateur (200) pour générer une composition parfumée ou aromatisée représentée par un identifiant numérique, **caractérisé en ce qu'**il comprend une étape de génération (115) d'une composition parfumée ou aromatisée en utilisant le dispositif auto-encodeur entraîné ou de réseau antagoniste génératif entraîné selon l'une quelconque des revendications 1 à 5.

7. Procédé mis en œuvre sur ordinateur (200) selon la revendication 6, dans lequel l'étape de génération (115) est configurée pour générer un identifiant numérique de composition parfumée ou aromatisée en fonction d'au moins une valeur représentative d'au moins une contrainte d'entrée pour des compositions générées représentatives d'au moins un paramètre hédonique, sensoriel et/ou physico-chimique, pour l'identifiant numérique de compositions parfumées ou aromatisées généré, parmi :
- une direction olfactive ou gustative associée à la composition parfumée ou aromatisée identifiée,
- une substance de conditionnement associée à la composition parfumée ou aromatisée identifiée,
- une valeur de stabilité ou de dégradation visuelle et/ou olfactive associée à la composition parfumée ou aromatisée identifiée,
- un pourcentage de biodégradabilité associé à la composition parfumée ou aromatisée identifiée,
- un pourcentage de carbone renouvelable associé à la composition parfumée ou aromatisée identifiée,
- une intensité psychophysique perçue associée à la composition parfumée ou aromatisée identifiée,
- une valeur de point d'éclair associée à la composition parfumée ou aromatisée identifiée,
- une valeur de toxicité associée à la composition parfumée ou aromatisée identifiée,
- une valeur de sensibilisation cutanée associée à la composition parfumée ou aromatisée identifiée,
- une valeur de compatibilité d'exhausteur associée à la composition parfumée ou aromatisée identifiée
- un nombre d'ingrédients dans la composition,
- une valeur d'accumulation environnementale associée à la composition parfumée ou aromatisée identifiée et/ou
- des indices de rétention pour la composition.

8. Procédé mis en œuvre sur ordinateur (200) selon la revendication 5 et l'une quelconque des revendications 6 ou 7, dans lequel l'étape (115) de génération est configurée pour générer un identifiant numérique de composition parfumée ou aromatisée associé à au moins un identifiant de substance de conditionnement secondaire en fonction d'une entrée d'au moins un identifiant numérique de composition parfumée ou aromatisée associé à un identifiant de substance de conditionnement primaire.

9. Procédé mis en œuvre sur ordinateur (300) selon l'une quelconque des revendications 1 à 7, lequel comprend :
- une étape (130) de génération d'un dispositif d'apprentissage automatique auxiliaire entraîné, comprenant :
- la fourniture (131) d'un ensemble original d'identifiants numériques de compositions parfumées ou aromatisées à titre d'exemples et d'étiquettes représentatives d'une caractéristique chimique, telle qu'une valeur de caractéristique olfactive ou une valeur représentative d'une quantité de composé chimique associée à ladite structure,
- l'entraînement (132) du dispositif d'apprentissage automatique auxiliaire en utilisant l'ensemble original d'identifiants numériques de compositions parfumées ou aromatisées à titre d'exemples pour fournir un classificateur de compositions et
- une étape (135) consistant à contraindre l'étape d'entraînement d'un dispositif de réseau antagoniste génératif ou d'un dispositif auto-encodeur avec le dispositif d'apprentissage automatique, ladite étape étant exécutée pendant ladite étape d'entraînement, sous forme de règle de renforcement, ou via le post-traitement de l'identifiant de composition parfumée ou aromatisée généré.

10. Procédé (100, 200, 300) selon l'une quelconque des revendications 1 à 9, qui comprend en outre une étape (136) de composition d'une composition parfumée ou aromatisée générée comprenant au moins deux ingrédients distincts.

11. Procédé (100, 200, 300) selon l'une quelconque des revendications 1 à 10, qui comprend en outre une étape (137) de sélection d'une composition parfumée ou aromatisée générée, comprenant au moins deux ingrédients distincts, à composer.

12. Dispositif auto-encodeur mis en œuvre sur ordinateur entraîné selon le procédé selon l'une quelconque des revendications 1 à 5.

13. Dispositif de réseau antagoniste génératif mis en œuvre sur ordinateur entraîné selon le procédé selon l'une quelconque des revendications 1 à 5.

14. Produit de programme informatique comprenant des instructions qui, lorsque le programme est exécuté par un ordinateur, amènent l'ordinateur à réaliser le procédé selon l'une quelconque des revendications 1 à 11.

15. Support de stockage lisible par ordinateur stockant des instructions qui, lorsqu'elles sont exécutées par un ordinateur, amènent l'ordinateur à effectuer les étapes selon l'une quelconque des revendications 1 à 11.

16. Dispositif (400) pour l'entraînement d'un dispositif réseau neuronal auto-encodeur ou de réseau antagoniste génératif à générer des représentations numériques indéterministes et réalistes de nouvelles compositions d'ingrédients parfumés ou aromatisés à composer, **caractérisé en ce qu'**il comprend :
- un moyen (405) de fourniture d'un ensemble original d'identifiants numériques de compositions parfumées ou aromatisées à titre d'exemples, lesdits identifiants numériques de compositions parfumées ou aromatisées à titre d'exemples étant représentatifs de compositions parfumées ou aromatisées matérialisées comprenant au moins deux ingrédients composés parfumés ou aromatisés distincts, l'ensemble original d'identifiants numériques de compositions parfumées ou aromatisées à titre d'exemples comprenant en outre, associée à au moins un identifiant numérique de composition parfumée ou aromatisée à titre d'exemple, une valeur représentative d'au moins un paramètre hédonique, sensoriel et/ou physico-chimique, et
- un moyen (410) d'entraînement d'un dispositif auto-encodeur ou dispositif de réseau antagoniste génératif en utilisant l'ensemble original d'identifiants numériques de compositions parfumées ou aromatisées à titre d'exemples pour générer un modèle génératif de compositions parfumées ou aromatisées entraîné pour générer des nouvelles compositions d'ingrédients parfumés ou aromatisés, comprenant au moins deux ingrédients composés parfumés ou aromatisés distincts, à composer, en fonction d'au moins une valeur représentative d'au moins une contrainte pour des compositions générées représentatives d'au moins un paramètre hédonique, sensoriel et/ou physico-chimique, pour la nouvelle composition parfumée ou aromatisée générée.

17. Dispositif (500) pour générer un identifiant numérique de composition parfumée ou aromatisée, **caractérisé en ce qu'**il comprend un moyen de génération (505) d'une composition parfumée ou aromatisée, comprenant au moins deux ingrédients distincts, en utilisant le dispositif auto-encodeur entraîné ou de réseau antagoniste génératif entraîné selon l'une quelconque des revendications 1 à 5.
